(19) Europäisches Patentamt / European Patent Office / Office européen des brevets

(11) **EP 4 122 886 B1**

(12) **EUROPEAN PATENT SPECIFICATION**

(45) Date of publication and mention
of the grant of the patent:
**03.09.2025 Bulletin 2025/36**

(21) Application number: **21771217.3**

(22) Date of filing: **16.03.2021**

(51) International Patent Classification (IPC):
*A61N 5/06* (2006.01)    *C01B 33/113* (2006.01)
*C09K 11/02* (2006.01)    *C09K 11/77* (2006.01)
*G01N 33/552* (2006.01)    *G01N 33/58* (2006.01)
*B82Y 5/00* (2011.01)    *B82Y 30/00* (2011.01)

(52) Cooperative Patent Classification (CPC):
**C09K 11/77342; A61N 5/062; C01B 33/113;
C09K 11/02; C09K 11/7732; G01N 33/552;
G01N 33/582; G01N 33/587; B82Y 5/00;
B82Y 30/00**

(86) International application number:
**PCT/JP2021/010645**

(87) International publication number:
**WO 2021/187492 (23.09.2021 Gazette 2021/38)**

(54) **COMPLEX BETWEEN SILICATE-BASED SUBSTRATE AND RARE EARTH ELEMENT COMPOUND, LIGHT-EMITTING NANO PARTICLES, CELL DETECTION METHOD, AND METHOD FOR PRODUCING COMPLEX BETWEEN SILICATE-BASED SUBSTRATE AND RARE EARTH ELEMENT COMPOUND**

KOMPLEX ZWISCHEN SILIKATBASIERTEM SUBSTRAT UND SELTENERDELEMENTVERBINDUNG, LICHTEMITTIERENDE NANOPARTIKEL, ZELLERKENNUNGSVERFAHREN, UND VERFAHREN ZUR HERSTELLUNG EINES KOMPLEXES ZWISCHEN SILIKATBASIERTEM SUBSTRAT UND SELTENERDELEMENTVERBINDUNG

COMPLEXE ENTRE UN SUBSTRAT À BASE DE SILICATE ET UN COMPOSÉ D'ÉLÉMENT DES TERRES RARES, NANOPARTICULES ÉLECTROLUMINESCENTES, MÉTHODE DE DÉTECTION DE CELLULE, ET MÉTHODE DE PRODUCTION D'UN COMPLEXE ENTRE UN SUBSTRAT À BASE DE SILICATE ET UN COMPOSÉ D'ÉLÉMENT DES TERRES RARES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.03.2020 JP 2020046605**

(43) Date of publication of application:
**25.01.2023 Bulletin 2023/04**

(73) Proprietors:
• **Ohara, Inc.
Sagamihara-shi, Kanagawa 252-5286 (JP)**
• **National University Corporation
Nagaoka University of Technology
Nagaoka-shi
Niigata 940-2188 (JP)**

• **National Institute of Technology
Hachioji-shi, Tokyo 1930834 (JP)**

(72) Inventors:
• **CHATANI, Sunao
Wakayama-shi, Wakayama 640-8404 (JP)**
• **INUI, Masahiko
Wakayama-shi, Wakayama 640-8404 (JP)**
• **TAGAYA, Motohiro
Nagaoka-shi, Niigata 940-2188 (JP)**
• **KATAOKA, Takuya
Nagaoka-shi, Niigata 940-2188 (JP)**
• **MOTOZUKA, Satoshi
Kitakyusyu-shi, Fukuoka 804-8550 (JP)**

**(Cont. next page)**

(74) Representative: **Maiwald GmbH**
**Elisenhof**
**Elisenstraße 3**
**80335 München (DE)**

(56) References cited:
**WO-A1-2008/123373** **CN-A- 107 311 217**
**JP-A- 2016 041 643** **JP-A- 2020 033 240**

- **HASSAN SYED MUJTABA UL ET AL: "Synthesis and characterization of sub-micrometer SiO2@NaYF4:Yb/Er beads and NaYF4:Yb/Er capsules for biomedical applications", ELECTROCHIMICA ACTA, vol. 183, 1 November 2015 (2015-11-01), AMSTERDAM, NL, pages 160 - 164, XP093130194, ISSN: 0013-4686, DOI: 10.1016/j.electacta.2015.04.051**

**Description**

TECHNICAL FIELD

The present invention relates to a light-emitting nanoparticle comprising a composite of a silicate-based base material and a rare-earth compound, a method for producing a composite of a silicate-based base material and a rare-earth compound, light-emitting nanoparticles, a cell detection method, and a medical device.

BACKGROUND ART

In recent years, there has been a demand for a technique of detecting tumors such as cancer cells or the like with high sensitivity at a cellular level by a marker material having high biocompatibility.

Patent Document 1 discloses, as a bioimaging material, a two-photon absorbing material consisting of a water soluble dendrimer in which a dye having a two-photon absorbing property and a fluorescence property and a dendron are bonded.

Patent Document 2 discloses use of a water-dispersible quantum dot in which a surface of the quantum dot is coated with a surfactant-type polymerization initiator including a hydrophobic group and a polar group, as a particle for in-vivo bioimaging.

Patent Document 3 discloses a production method of a bioimaging nanoparticle including producing a hydrophobic nanoparticle which maintains individual dispersibility in a nonpolar organic solvent, wherein in a hydrophobic inorganic nanoparticle having a core or core/shell structure protected by a surfactant, the surfactant is partially substituted by adding 1 to 30 equivalents of an organic ligand wherein a thiol group and a hydrophilic group are bonded by a hydrocarbon chain having a carbon number of 8 to 20, and the surface of the nanoparticles is surface-modified so that only one part is hydrophilic, by forming a metal thiolate (M-S) bond, and the like.

Patent Document 4 discloses a fluorescent particle used for bioimaging, which is a fluorescent particle having upconversion characteristics as a phenomenon of obtaining visible light fluorescence with the use of a low energy light such as an infrared light or the like as an excitation light, and the material of the fluorescent particles is one material, or a combination of two or more materials among $Y_2O_3:Er^{3+}$, $Yb^{3+}$, $Y_2O_3:Er^{3+}$, $NaYF_4:Er^{3+}$, and $Yb^{3+}$.

Patent Document 5 discloses a semiconductor nanoparticle to be used for molecule/cell imaging, which is a semiconductor nanoparticle having an average particle size of 1 to 20 nm, containing an atom pair of a main component atom constituting the same, and an atom pair of the corresponding differing atom or a differing atom having an equivalent valence electron arrangement, and further the dopant distributed on the semiconductor nanoparticle surface or in the vicinity thereof.

Patent Document 6 discloses a fluorescent labeling agent for pathological diagnosis including fluorescent material encapsulated nanoparticles including a first fluorescent substance, and a second fluorescent substance having a distinguishable excitation/light-emitting property from the first fluorescent substance.

Patent Document 7 discloses a fluorescent labeling agent including a rare-earth fluorescent complex-containing silica particle containing rare-earth fluorescent complex, and a target molecule measurement kit.

Furthermore, Non-Patent Documents 1 to 4 also disclose use of quantum dots, fluorescent dyes, or the like, for bioimaging. Hassan Syed Mujtaba, et al., disclose in Electrochimica Acta 2015, 183, pp. 160-164, the synthesis and characterization of sub-micrometer $SiO_2@NaYF_4:Yb/Er$ beads and $NaYF_4:Yb/Er$ capsules for biomedical applications.

As described above, in recent years, there has been a demand for a technique of detecting tumors such as cancer cells with high sensitivity at the cellular level by a marker material having high biocompatibility. For example, in the cancerization of a cell, before a morphological change occurs, an activity change at the molecular level occurs. For example, a cancer cell tends to consume a large amount of glucose compared with a normal cell. At the same time, folate receptors are overexpressed on the cell membrane, resulting in tendency of specifically binding to / taking up folic acid molecules. High sensitivity imaging of such changes at the molecular level of a cell would make it possible to realize very early stage detection of cancer cells and the like.

However, in the case of using an organic molecule as an imaging material for imaging, the rate of degradation/fading is high, for example, there has been the problem that light-emitting particles become quenched or the like by photoirradiation after several tens of minutes under fluorescence observation. Furthermore, in the case of using an inorganic material such as quantum dots as a bioimaging material, highly toxic elements such as cadmium may be included, and there have been problems with biocompatibility and the like.

Thus, the present inventors created a composite particle wherein a light-emitting molecule or ion is included in an inorganic material, and developed a light-emitting nanoparticle provided with light emitting stability and light resistance, and having low biological toxicity (Patent Document 8).

Patent Document 1: Japanese Unexamined Patent Application, Publication No. 2010-133887
Patent Document 2: Japanese Unexamined Patent Application, Publication No. 2009-190976
Patent Document 3: Japanese Unexamined Patent Application, Publication No. 2009-107106
Patent Document 4: Japanese Unexamined Patent Application, Publication No. 2013-14651
Patent Document 5: PCT International Publication No. WO2009/066548
Patent Document 6: Japanese Unexamined Patent Application, Publication No. 2013-57037
Patent Document 7: PCT International Publication No. WO2005/023961
Patent Document 8: PCT International Publication No. WO2017/170531

Non-Patent Document 1: "Selective molecular imaging of viable cancer cells with pH-activatable fluorescence probes" Nature Medicine 15, 104 (2009)
Non-Patent Document 2: "Quantum Dot Bioconjugates for Ultrasensitive Nonisotopic Detection." Science 281, 2016 (1998) Non-Patent Document 3: "Nucleic Acid-Passivated Semiconductor Nanocrystals: Biomolecular Templating of Form and Function." Accounts of Chemical Research, 43, 173 (2010)
Non-Patent Document 4: "Multimodal-Luminescence Core-Shell Nanocomposites for Targeted Imaging of Tumor Cells." Chem. Eur. J., 15, 3577 (2009)

DISCLOSURE OF THE INVENTION

Problems to be Solved by the Invention

In order to achieve bioimaging with high sensitivity, light-emitting intensity of light-emitting particles is desirably high. However, in Patent Document 8, light-emitting molecules/ions may agglomerate, or the amount of light-emitting molecules is small and the light-emitting intensity may be degraded. Note here that high light-emitting intensity is similarly demanded for applications other than bioimaging, and the problem that when light-emitting molecules/ions agglomerate or the amount of light-emitting substance is small, the light-emitting intensity may be degraded similarly occurs in applications other than bioimaging.

The present invention has been made in view of the above-mentioned problem, and has an object to provide a light-emitting nanoparticle comprising a composite of a silicate-based base material and a rare-earth compound, having high light-emitting intensity, and light-emitting nanoparticle including the same, a cell detection method, a medical device, and a method for producing the composite of a silicate-based base material and a rare-earth compound.

Means for Solving the Problems

The present inventors have completed the present invention based on the finding that when a composite of a silicate-based base material and a rare-earth compound includes the silicate-based base material containing elemental silicon (Si) and elemental oxygen (O) and the rare-earth compound, the rare-earth compound includes at least one selected from a chloride of a rare-earth element and a fluoride of a rare-earth element, and the silicate-based base material has a solid $^{29}$Si-NMR spectrum satisfying $Q_4/Q_3$ of 1.6 to 3.9 where $Q_4$ represents a peak area derived from $Si(OSi)_4$ and $Q_3$ represents a peak area derived from $HO\text{-}Si(OSi)_3$, an amount of a light-emitting rare-earth element such as Eu is sufficient, agglomeration of light-emitting rare-earth ions is suppressed, and the light-emitting intensity is high. In other words.

The present invention relates to a light-emitting nanoparticle comprising a composite of a silicate-based base material and a rare-earth compound, the silicate-based base material containing elemental silicon (Si) and elemental oxygen (O), wherein the silicate-based base material is a powder having an average particle diameter of 50 nm or more and 470 nm or less, and the rare-earth compound is a light-emitting substance,

in which the rare-earth compound includes at least one selected from a chloride of a rare-earth element and a fluoride of a rare-earth element, and
the silicate-based base material has a solid $^{29}$Si-NMR spectrum satisfying $Q_4/Q_3$ of 1.6 to 3.9 where $Q_4$ represents a peak area derived from $Si(OSi)_4$ and $Q_3$ represents a peak area derived from $HO\text{-}Si(OSi)_3$.

Furthermore, the present invention relates to the use of the light-emitting nanoparticle as described above for bioimaging.

In addition, the present invention relates to a cell detection method, including allowing the light-emitting nanoparticle as described above to be taken into a cell, irradiating the light-emitting nanoparticle with light, and observing the cell.

The present specification further describes, without being claimed, a treating method for treating a non-human animal, the method including administering the light-emitting nanoparticle as described above to the animal, irradiating the light-emitting nanoparticle with light, and treating the animal.

Moreover, the present invention relates to a medical device including a testing portion for carrying out testing of an internal cell, a diagnosis portion for carrying out diagnosis of the internal cell, and/or a treatment portion for carrying out treatment of the internal cell, and further including a light irradiating portion for introducing the light-emitting nanoparticle as described above into an internal cell and irradiating the light-emitting nanoparticle with light, when the testing, the diagnosis, and/or the treatment are carried out.

The present invention also relates to a method for producing the composite of a silicate-based base material and a rare-earth compound as defined above, the method including:

mixing a silicate-based base material and a raw material of a rare-earth compound with each other to carry out a solid-phase mechanochemical reaction,

in which the silicate-based base material has a solid $^{29}$Si-NMR spectrum satisfying $Q_4/Q_3$ of 1.6 to 3.9 where $Q_4$ represents a peak area derived from $Si(OSi)_4$ and $Q_3$ represents a peak area derived from $HO-Si(OSi)_3$.

in addition, the present invention relates to a method for producing the composite of a silicate-based base material and a rare-earth compound as defined above, the method including:

dissolving a surface of the silicate-based base material in a basic atmosphere, and

reacting the silicate-based base material in which the surface is dissolved with a raw material of the rare-earth compound, in which the silicate-based base material has a solid $^{29}$Si-NMR spectrum satisfying $Q_4/Q_3$ of 1.6 to 3.9 where $Q_4$ represents a peak area derived from $Si(OSi)_4$ and $Q_3$ represents a peak area derived from $HO-Si(OSi)_3$.

in addition, the present invention relates to a mixed crystal including at least one compound selected from a compound represented by the following chemical formula (5) and a compound represented by the following chemical formula (6), and amorphous silica, which is a powder having an average particle diameter of 50 nm or more and 470 nm or less, the mixed crystal having a powder X-ray diffraction pattern having a fourth diffraction peak in a diffraction angle ($2\theta$) range of 26.6° to 28.6°, a fifth diffraction peak in a diffraction angle ($2\theta$) range of 44.8° to 46.8°, and a sixth diffraction peak in a diffraction angle ($2\theta$) range of 24.3° to 26.3°, in which a half value width of the fourth diffraction peak is 0.3° or less, a half value width of the fifth diffraction peak is 0.57° or less, and a half value width of the sixth diffraction peak is 0.22° or less, and/or having the fourth diffraction peak, the fifth diffraction peak, and a seventh diffraction peak in a diffraction angle ($2\theta$) range of 30.8° to 32.8°, in which a half value width of the fourth diffraction peak is 0.3° or less, a half value width of the fifth diffraction peak is 0.57° or less, and a half value width of the seventh diffraction peak is 0.38° or less.

$$EuOF \qquad (5)$$

$$EuF_3 \qquad (6)$$

Effects of the Invention

The present invention can provide a light-emitting nanoparticle comprising a composite of a silicate-based base material and a rare-earth compound having high light-emitting intensity, a cell detection method, a method for treating an animal, a medical device, and a method for producing a composite of a silicate-based base material and a rare-earth compound.

BRIEF DESCRIPTION OF THE DRAWINGS

FIG. 1 is a schematic sectional view showing an example of a composite of a silicate-based base material and a rare-earth compound;

FIG. 2 shows a solid $^{29}$Si-NMR spectrum of the silicate-based base material;

FIG. 3 shows a powder X-ray diffraction pattern of the silicate-based base material;

FIG. 4A and 4B show measurement results of an average particle size by a field emission type scanning electron microscope (FE-SEM) of a silicate-based base material;

FIG. 5 is a schematic side view for illustrating a production device used for a production method by a mechanochemical method;

FIG. 6 shows powder X-ray diffraction patterns of particles of Examples and Comparative Examples;

FIG. 7A and 7B show excitation spectrums and fluorescence spectrums of particles of Examples;

FIG. 8A and 8B show transmission electron microscope (TEM) observation images of particles of Examples;

FIG. 9A and 9B show calculation results of average particle sizes of the particles of Examples by a field emission type scanning electron microscope (FE-SEM) observation;

FIG. 10 shows powder X-ray diffraction patterns of particles of Examples;

FIG. 11 is a view showing measurement results of cell densities of Examples and Comparative Examples;

FIG. 12 is a view showing measurement results of fluorescence densities (PL) of Examples and Comparative Examples;

FIG. 13 is a view showing measurement results of fluorescence densities (PL) of Examples and Comparative Examples;

FIG. 14 is a schematic side view for illustrating a production method by gas phase method;

FIG. 15 shows powder X-ray diffraction patterns of particles of Examples;

FIG. 16 shows powder X-ray diffraction patterns of particles of Examples and Comparative Examples; and

FIG. 17 shows fluorescence spectrum of particles of Examples.

PREFERRED MODE FOR CARRYING OUT THE INVENTION

Hereinafter, the present invention will be described in more detail.

<Composite of silicate-based base material and rare-earth compound>

A composite of a silicate-based base material and a rare-earth compound of the present invention includes a silicate-based base material containing elemental silicon (Si) and elemental oxygen (O), and a rare-earth compound. The rare-earth compound includes at least one selected from chloride of a rare-earth element and fluoride of a rare-earth element. Furthermore, the silicate-based base material has a solid $^{29}$Si-NMR spectrum satisfying $Q_4/Q_3$ of 1.6 to 3.9 where $Q_4$ represents a peak area derived from $Si(OSi)_4$ and $Q_3$ represents a peak area derived from $HO\text{-}Si(OSi)_3$.

In this way, a composite of a specific silicate-based base material and a rare-earth compound including chloride of the rare-earth element or fluoride of the rare-earth element, as shown in the below-mentioned Examples, agglomeration of rare-earth element ions as light-emitting substance is suppressed and a supporting amount of the rare-earth element into the silicate-based base material becomes appropriate, fluorescence intensity (light-emitting intensity) becomes high. On the other hand, when $Q_4/Q_3$ is less than 1.6, since the supporting amount of the rare-earth element is large, agglomeration occurs and fluorescence intensity is deteriorated. Furthermore, when $Q_4/Q_3$ is more than 3.9, since the supporting amount of the compound of the rare-earth element is small, the fluorescence intensity is deteriorated.

The "composite of a silicate-based base material and a rare-earth compound" is not a simple mixture obtained by mixing a silicate-based base material and a rare-earth compound, but is a structure body having a rare-earth compound, for example, on a surface of the silicate-based base material, and a structure body in which a silanol group of a silicate-based base material and an oxygen atom of a siloxane bond, and rare-earth ions constituting the rare-earth compound are chemically bonded one another by, for example, a coordination bond.

The rare-earth ions constituting the rare-earth compound are not particularly limited, but are preferably at least one type selected from the group consisting of trivalent Ce, tetravalent Ce, trivalent Pr, trivalent Nd, trivalent Pm, trivalent Sm, divalent Eu, trivalent Eu, trivalent Gd, trivalent Tb, trivalent Dy, trivalent Ho, trivalent Er, trivalent Tm, trivalent Yb, trivalent Lu, and trivalent Tb. Any of these are a light-emitting substance. Among these, $Eu^{3+}$ that is trivalent Eu is preferable. This is preferable because $Eu^{3+}$ is excited and emits light in the visible light region. For example, a crystal of a europium compound excites and emits light in a visible light region, and the excitation wavelength $\lambda_{ex}$ is, for example, 395 nm and 464 nm, and the fluorescence wavelength $\lambda_{em}$ is, for example, 615 nm.

The rare-earth compound may be an amorphous, but is preferably a crystal. When the rare-earth compound is a crystal, as compared with an amorphous, elusion of ions such as a chloride ion and a fluoride ion does not easily occur, so that an adverse effect by the elusion of ions is suppressed. Therefore, cytotoxicity is low, and can be preferably used for bioimaging.

The silicate-based base material contains elemental silicon (Si) and elemental oxygen (O). The molar ratio (O/Si) that is a ratio of O to Si in the silicate-based base material, is preferably 2.0 to 2.2. The silicate-based base material is a material as a skeleton of the composite of the silicate-based base material and the rare-earth compound. Examples of the silicate-based base material include silicon oxide such as silica, a base material including silicate. These may be a crystal, but is preferably an amorphous from the viewpoint of biotoxicity.

Furthermore, the silicate-based base material has a solid $^{29}$Si-NMR spectrum satisfying $Q_4/Q_3$ of 1.6 to 3.9 where $Q_4$ represents a peak area derived from $Si(OSi)_4$ and $Q_3$ represents a peak area derived from $HO\text{-}Si(OSi)_3$. The

$Q_4/Q_3$ is preferably 2.0 to 3.9, and more preferably 2.2 to 2.6.

Such a silicate-based base material containing elemental silicon (Si) and elemental oxygen (O) and satisfying $Q_4/Q_3$ of 1.6 to 3.9 can be obtained as a particulate substance (a soot body or a by-product) generated in a method for producing silica glass by a conventionally known soot method (for example, a production method of silica glass by Vapor phase Axial Deposition (VAD)). For example, when silica glass is produced as porous synthetic quartz glass (soot body) by hydrolyzing silicon tetrachloride in an oxygen-hydrogen flame using silicon tetrachloride as a raw material, particles which are hydrolyzed in the oxygen-hydrogen flame and then desorbed from the flame and do not become porous synthetic quartz glass can be the silicate-based base material. By adjusting the above conditions, the above silicate-based base material is obtained. For example, silicon tetrachloride as a raw material is introduced into the central portion of the oxygen-hydrogen flame burner to adjust a flame temperature zone length and gas balance of oxygen-hydrogen gas. Specifically, nucleation and grain growth are adjusted, for example, in a range of the flame temperature zone length at 1000°C or more: 100 mm or more and 800 mm or less, and volume ratio of hydrogen to oxygen ($H_2/O_2$): 1.0 or more and 2.5 or less. As the flame temperature zone length is increased, $Q_4/Q_3$ tends to increase, and as $H_2/O_2$ increases, $Q_4/Q_3$ tends to decrease.

The shape of the silicate-based base material is not particularly limited. The shape may be, for example, a spherical shape (powdery) or may be a plate shape, but the shape is preferably spherical shape because the cytotoxicity is low. Also, the size of the silicate-based base material is not particularly limited. When the composite of the silicate-based base material and the rare-earth compound is used for bioimaging, from the viewpoint of cytotoxicity, it is preferable that the silicate-based base material has a spherical shape and an average particle size of 50 nm or more and 470 nm or less, and is amorphous.

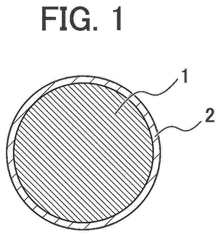

When the silicate-based base material has a spherical shape, as shown in a schematic sectional view of FIG. 1 showing an example of the composite of the silicate-based base material and the rare-earth compound, the composite of the silicate-based base material and the rare-earth compound (hereinafter, also referred to as "composite") is a structure body in which a surface of the silicate-based base material 1 is covered with the rare-earth compound 2. Furthermore, when a silicate-based base material having a plate shape is used, a composite in which at least a part of the surface of the silicate-based base material of the plate shape is covered with the rare-earth compound 2 is obtained. The thickness of the layer including the rare-earth compound 2 covering the silicate-based base material 1 is not particularly limited, but the thickness is, for example, 5 nm or more and 25 nm or less, and preferably, 10 nm or more and 20 nm or less. Note here that FIG. 1 shows a composite in which an entire surface of the silicate-based base material 1 having a spherical shape is covered with the rare-earth compound 2, but the rare-earth compound 2 is only required to cover at least a part of the surface of the silicate-based base material 1.

In the composite, a percentage of the number of moles of the rare-earth element with respect to a total number of moles of the elemental silicon and the rare-earth element (number of moles of rare-earth element / (number of moles of rare-earth element + number of moles of Si)) is preferably 0.1 mol% or more and 7 mol% or less, and more preferably 4 mol% or more and 7 mol% or less.

When the rare-earth compound includes the chloride of the rare-earth element, the rare-earth compound includes, for example, compounds represented by the following chemical formulae (1), (3) and (4).

$$EuCl_x \qquad (1)$$

$$Eu(OH)_2Cl \qquad (3)$$

$$EuOCl \qquad (4)$$

(In the formula (1), x is 0.05 or more and 5 or less.)

Furthermore, it is preferable that the compounds represented by the above chemical formulae (1), (3) and (4) have a powder X-ray diffraction pattern having a first diffraction peak in a diffraction angle ($2\theta$) range of 34.3° to 36.1° in which a half value width (half width) of the first diffraction peak is 1.8° or less, and/or having a second diffraction peak in a diffraction angle ($2\theta$) range of 28.6° to 29.6° and a third diffraction peak in a diffraction angle ($2\theta$) range of 36.8° to 38.4° in which a half value width of the second diffraction peak is 1.0° or less and a half value width of the third diffraction peak is 1.6° or less. The compounds represented by the chemical formulae (1) to (4) may have a first diffraction peak in a diffraction angle ($2\theta$) range of 34.3° to 36.1° in which a half value width of the first diffraction peak is 1.8° or less; may have a second diffraction peak in a diffraction angle ($2\theta$) range of 28.6° to 29.6° and a third diffraction peak in a diffraction angle ($2\theta$) range of 36.8° to 38.4° in which a half value width of the second diffraction peak is 1.0° or less and a half value width of the third diffraction peak is 1.6° or less; and also may have a first diffraction peak in a diffraction angle ($2\theta$) range of 34.3° to 36.1° in which a half value width of the first diffraction peak is 1.8° or less, a second diffraction peak in a diffraction angle ($2\theta$) range of 28.6° to 29.6° and a third diffraction peak in a diffraction angle ($2\theta$) range of 36.8° to 38.4° in which a half value width of the second diffraction peak is 1.0° or less and a half value width of the third diffraction peak is 1.6° or less. The half value width of the first diffraction peak is preferably 1.1° or less. The half value width of the second diffraction peak is preferably 0.6° or less. The half value width of the third diffraction peak is preferably 1.0° or less. The first diffraction peak is derived from a crystal of the compound represented by the chemical formula (1). The second diffraction peak and the third diffraction peak are derived from a crystal of the compound represented by the chemical formula (3) or (4). In the chemical formula (1), x is preferably 0.2 or more and 0.6 or less.

The description "having a diffraction peak in a diffraction angle ($2\theta$) range of a° to b°'" means that a peak top position of the diffraction peak (a diffraction peak top position) is within a range of a° to b°. Therefore, for example, in a broad peak, all of the peaks from an end portion to another end portion are not necessarily required to be included within a range of a° to b°.

Furthermore, the crystal bodies of the compounds represented by the chemical formulae (1), (3) and (4) may have a different peak other than the above specific diffraction peak. For example, the compound represented by the chemical formula (1) may also have a diffraction peak in a diffraction angle ($2\theta$) range of 25.9° to 26.5° and a diffraction peak in a diffraction angle ($2\theta$) range of 31.6° to 32.2°. The diffraction peak in a diffraction angle ($2\theta$) range of 25.9° to 26.5° and the diffraction peak in a diffraction angle ($2\theta$) range of 31.6° to 32.2° respectively have preferably a half value width of 0.6° or less, and further preferably 0.4° or less. Furthermore, the crystal bodies of the compound represented by the chemical formula (3) or (4) may also have a diffraction peak in a diffraction angle ($2\theta$) range of 39.0° to 40.2°. The half value width of the diffraction peak in a diffraction angle ($2\theta$) range of 39.0° to 40.2° is preferably 1.2° or less, and further preferably 0.8° or less.

Conventionally, crystal bodies of the compounds represented by the above chemical formulae (1), (3) and (4) have not been known. However, as will be described in detail later, when a specific silicate-based base material and europium chloride (III) hexahydrate are subjected to a solid-phase mechanochemical reaction under specific conditions, crystals of compounds represented by chemical formulae (1), (3) and (4) having the specific diffraction peaks can be obtained. Note here that the "crystal" means a substance not being amorphous, and a substance having crystallinity represented by the following formula (7) of more than 0. The crystallinity is preferably more than 0.10.

$$\text{Crystallinity} = \{\text{crystal diffraction peak area / amorphous halo diffraction peak area}\} \qquad (7)$$

(In the formula (7), the "crystal diffraction peak area" is a sum of the areas of the diffraction peaks derived from the crystal in $2\theta = 20°$ to $55°$; the "amorphous halo diffraction peak area" is a value obtained by subtracting the crystal diffraction peak area from the sum of the areas of all the diffraction peaks observed in $2\theta = 20°$ to $55°$.)

When the rare-earth compound includes a fluoride of a rare-earth element, the rare-earth compound includes, for example, a compound represented by chemical formula (5) or (6). A mixed crystal of at least one compound selected from compounds represented by chemical formulae (5) and (6) and silica derived from a silicate-based base material may be used.

$$\text{EuOF} \qquad (5)$$

$$\text{EuF}_3 \qquad (6)$$

Furthermore, it is preferable that the compound represented by the above chemical formula (5) or (6) has a powder X-ray diffraction pattern having a fourth diffraction peak in a diffraction angle ($2\theta$) range of 26.6° to 28.6°, a fifth diffraction peak in a diffraction angle ($2\theta$) range of 44.8° to 46.8°, and sixth diffraction peak in a diffraction angle ($2\theta$) range of 24.3° to 26.3, in which a half value width of the fourth diffraction peak is 0.3° or less, a half value width of the fifth diffraction peak is 0.57° or less, a half value width of the sixth diffraction peak is 0.22° or less, and/or and having the fourth diffraction peak, the fifth diffraction peak, and a seventh diffraction peak in a the diffraction angle ($2\theta$) range of 30.8° to 32.8°, in which a half value width of the fourth diffraction peak is 0.3° or less, a half value width of the fifth diffraction peak is 0.57° or less, and a half value width of the seventh diffraction peak is 0.38° or less. The fourth diffraction peak, the fifth diffraction peak, and the seventh diffraction peak are derived from the crystal of the compound represented by the formula (5). Furthermore, the fourth diffraction peak, the fifth diffraction peak, and the sixth diffraction peak are derived from the crystal of the compound represented by the formula (6).

The rare-earth compound may be, for example, terbium chloride ($TbCl_3$), and terbium fluoride ($TbF_3$).

Note here that in the present invention, the rare-earth compound may have a different peak other than the above-specified diffraction peak.

The composite of the silicate-based base material and the rare-earth compound of the present invention preferably includes a molecule containing a carbon element on a surface thereof. The amount of molecule containing a carbon element is not particularly limited, but, for example, the percentage of the number of moles of carbon atoms with respect to the number of moles of silicon atoms included in the composite of the silicate-based base material and the rare-earth compound (number of moles of C / number of moles of Si) is preferably 0.05 mol% to 160 mol%, and more preferably 6 mol% to 9 mol%.

<Method for producing composite of silicate-based base material and rare-earth compound>

A composite of the silicate-based base material and the rare-earth compound of the present invention can be produced by a production method including mixing a silicate-based base material and a raw material of a rare-earth compound with each other to carry out a solid-phase mechanochemical reaction, in which the silicate-based base material has a solid $^{29}$Si-NMR spectrum satisfying $Q_4/Q_3$ of 1.6 to 3.9 where $Q_4$ represents a peak area derived from $Si(OSi)_4$ and $Q_3$ represents a peak area derived from $HO\text{-}Si(OSi)_3$.

The silicate-based base material is described in the above-mentioned <Composite of the silicate-based base material and rare-earth compound>.

Examples of the raw material of the rare-earth compound include europium chloride (III) hexahydrate, europium chloride (III) anhydride in a case where the rare-earth compound is chloride of the rare-earth element, and include europium fluoride (III) hexahydrate and europium fluoride (III) anhydride in a case where the rare-earth compound is fluoride of the rare-earth element.

Such a silicate-based base material and a raw material of a rare-earth compound are mixed and subjected to a solid-phase mechanochemical reaction under a load (for example, under a load of 2 N or more and 24 N or less, and preferably 2 N or more and 12 N or less). A method for subjecting a silicate-based base material and a raw material of a rare-earth compound to a solid-phase mechanochemical reaction is not particularly limited. For example, a powdery silicate-based base material and a raw material of a rare-earth compound may be placed in a mortar, and pulverized by rotating a pestle while applying a load of 2 N or more and 24 N or less to the pestle. When the above operation is carried out with the mortar mounted on the electronic balance (electronic scale), the load value can be read by the metric indicator of the electronic balance. By increasing the load to be loaded, minute particulate composite is produced. The silicate-based base material and the rare-earth compound are composed (bonded) at a load of 2 N or more, and the rare-earth compound starts to be crystalized at a load of 4 N or more. By increasing the load, crystallinity can be enhanced. Note here that when the load exceeds 12 N, peeling of the rare-earth compound (light-emitting layer) accompanying pulverization tends to progress to cause phase separation and refinement (less than 50 nm). Therefore, the load is preferably 12 N or less. Furthermore, pulverization may be carried out by mounting europium chloride (III) on a surface of the plate-shaped silicate-based base material and moving the pestle while applying a load of 4 N or more and 24 N or less, and preferably 2 N or more and 12 N or less. In this case, by carrying out the above-mentioned operation while a plate-shaped silicate-based base material is mounted on an electronic balance, the load value can be read with a metric indicator of the electronic balance.

The percentage of the silicate-based base material and the raw material of the earth compound is not particularly limited, but the percentage of the number of moles of the rare-earth element with respect to the total number of moles of Si and the rare-earth element (number of moles of rare-earth element) / (number of moles of rare-earth element + number of moles of Si)) is preferably 0.1 mol% or more and 7.0 mol% or less. When europium chloride (III) hexahydrate is used as the raw material of the rare-earth compound, when the amount of Eu with respect to Si is increased, the compound represented by the chemical formula (3) tends to be generated through the compound represented by the chemical formula (4). Furthermore, when europium fluoride (III) hexahydrate is used as the raw material of the rare-earth compound, the compound represented by the chemical formula (5) is generated when the amount of Eu with respect to Si is small, and when the amount of Eu with respect to Si is increased, the compound represented by the chemical formula (5) is generated and at the same time, the compound represented by the chemical formula (6) tends to remain.

In the solid-phase mechanochemical reaction using the silicate-based base material satisfying $Q_4/Q_3$ of 2.0 to 3.9, europium chloride (III) hexahydrate is added such that the percentage of the number of moles of the europium element with respect to the total number of moles of elemental silicon and a europium element is 1.0 mol% or more and, and the above reaction is carried out under a load at 4 N or more and 24 N or less. Thus, a composite of the crystal of a europium compound and the silicate-based base material can be produced. The percentage of the number of moles of the europium element with respect to the total number of moles of the elemental silicon and a europium element is preferably 1.0 mol% or more and 7.0 mol% or less.

The europium compound is conventionally difficult to be made separately, but according to the above-mentioned production method, the compounds represented by chemical formulae (1), (3) and (4) can be separated based on the amount of Eu in the solid-phase mechanochemical reaction. Specifically, in a state in which the concentration of europium during a solid-phase mechanochemical reaction is low (in a state in which OH during the reaction is small in amount), the compound represented by the chemical formula (1) coexists with a compound represented by the chemical formulae formula (2): $Eu(OH)_2$, and when a multilayer structure starts to be formed according to increase in concentration of europium during reaction, the compound represented by the chemical formula (1) and the compound represented by the chemical formula (2) interact with each other, and tend to be changed into the compounds represented by the chemical formulae (3) and (4). Therefore, the compounds represented by the chemical formulae (1), (3) and (4) can be made separately based on the amount of Eu in the solid-phase mechanochemical reaction.

Furthermore, in the solid-phase mechanochemical reaction using the silicate-based base material satisfying $Q_4/Q_3$ of 2.0 to 3.9, europium fluoride is added such that the percentage of the number of moles of the europium element with respect to the total number of moles of elemental silicon and a europium element is 1.0 mol% or more and, and the above reaction is carried out under a load at 4 N or more and 24 N or less. Thus, a composite of the crystal of a europium compound and the silicate-based base material can be produced.

A solid-phase mechanochemical reaction is carried out, and then baking is carried out, and then washing with organic solvent or water is carried out as necessary.

With this production method, the rare-earth compound is formed on a surface of the silicate-based base material. For example, when the spherical-shaped silicate-based base material is used, a composite having a rare-earth compound covering at least a part of the surface of the spherical-shaped silicate-based base material is obtained. It is inferred that the rare-earth compound strongly binds to the silicate-based base material by some kind of chemical bonds such as a coordination bond between oxygen atoms existing on the surface of the silicate-based base material and the rare-earth atoms contained in the rare-earth compound.

The above-mentioned composite of the silicate-based base material and the rare-earth compound can be produced also by the gas phase method. Specifically, the composite of the silicate-based base material and the rare-earth compound can be produced by a production method including a step of dissolving the surface of the silicate-based base material in an basic atmosphere, and a step of reacting the silicate-based base material having a surface dissolved with the raw material of the rare-earth compound, in which the silicate-based base material has a solid $^{29}$Si-NMR spectrum satisfying $Q_4/Q_3$ of 1.6 to 3.9 where $Q_4$ represents a peak area derived from $Si(OSi)_4$ and $Q_3$ represents a peak area derived from $HO\text{-}Si(OSi)_3$.

Firstly, the surface of a silicate-based base material is dissolved in a basic atmosphere. The reactivity with the raw material of the rare-earth compound can be enhanced by dissolving the surface of the silicate-based base material in a basic atmosphere. Examples of the basic atmosphere include an atmosphere including ammonia vapor. The silicate-based base material is similar to that described for the solid phase mechanical method. In the case where the silicate-based base material is spherical (powdery), the surface of the silicate-based base material may be dissolved by mixing and pulverizing the silicate-based base material and the raw material of the rare-earth compound, and placing the obtained mixed and pulverized product in a basic atmosphere.

Next, a silicate-based base material having a surface that has been dissolved is allowed to react with the raw material of the rare-earth compound. Thus, the silicate-based base material such as silica that has been dissolved is deposited again and $Eu^{3+}$ coprecipitates. Thereby, a crystal of the rare-earth compound and $NH_4Cl$ is formed on the surface of the silicate-based base material. The raw material of the rare-earth compound is the same as those described for the solid phase mechanical method. After the reaction is carried out by a gas phase method, baking is carried out, and washing with organic solvent or water may be carried out as necessary.

Note here that as described above, without using a surfactant, a composite in which agglomeration of the rare-earth ions is suppressed can be produced.

<Use of light-emitting nanoparticle and light-emitting nanoparticle>

A composite of a silicate-based base material and a rare-earth compound of the present invention has low cytotoxicity, and has the rare-earth compound formed on the surface of the silicate-based base material functioning as a light-emitting substance, so that the composite can be preferably used for bioimaging technique. For example, the composite of the silicate-based base material and the rare-earth compound of the present invention can be used as the light-emitting nanoparticle for the bioimaging technique.

The average particle size of the light-emitting nanoparticle is 50 nm to 470 nm. By making the average particle size within this range, it becomes easy for the particles to be taken up by the target cells, and also tends to be suitable in observing the cells. On the other hand, it is not preferable that the average particle size is small because there is a tendency to cause toxicity problems by acting on the active function of the cell. The average particle size of the light-emitting nanoparticle is preferably 100 nm to 400 nm. Note here that in the case where the light-emitting nanoparticles are not used, for example, for cell imaging, the average particle size may be larger than 470 nm.

The light-emitting nanoparticle to be used for bioimaging technique preferably includes a hydroxyl group (OH group) on the surface thereof. Also preferably, the surface of the light-emitting nanoparticle is modified by an amino group, and, for example, may also be formed using a silane coupling agent containing an amino group. When silicate-based base material has pores, the OH group and the amino group may be on the inner surface of the pores, but preferably on the outer surface of the pores. When the OH group or the amino group is fixed by a hydrogen bond or a covalent bond by a condensation polymerization to the cell binding molecule, and the surface of the light-emitting nanoparticle is modified by the cell binding molecule, the cell binding molecule can specifically bind to a cancer cell or a normal cell. When the cell binding molecule specifically binds to a cell, the light-emitting nanoparticle is taken up into the cell. Thus, the light-emitting nanoparticle is made to emit light inside the cell, and a cancer cell or the like can be detected.

Examples of the cell binding molecule include an HER2 antibody, antibodies specifically binding to human epidermal growth factor receptor, cancer-specific antibodies, phosphorylation protein antibodies, folic acid, antibodies specifically binding to folic acid receptor β, vascular endothelial cell-specific antibodies, tissue-specific antibodies, transferrin, transferrin-bonding peptide, proteins having affinity to sugar chains, and the like. Among these, folic acid, having a tendency to be taken up by cancer cells, is preferably used as the cell binding molecule. Folic acid receptors are over-expressed on cell membranes for cancer cells, resulting in a tendency of selectively binding to and taking up folic acid molecules.

Furthermore, the surface of the light-emitting nanoparticle may be modified by an anticancer agent molecule. When the anticancer agent molecule specifically binds to the cancer cell, the light-emitting nanoparticle will be taken up into the cell. In this way, the light-emitting nanoparticle inside the cell is allowed to emit light, and can detect cancer cells. Further, the anticancer agent is also taken up by the cell, and the anticancer agent molecule can act, and the proliferation of cancer cells can be suppressed.

The cell binding molecule or anticancer agent molecule are preferably modified and fixed to the surface of the light-emitting nanoparticle by a chemical bond. Examples of the chemical bond include a peptide bond (-CO-NH-), a hydrogen bond, or the like.

The excitation wavelength and the light-emission wavelength of the light-emitting nanoparticles are preferably in the visible light region. When the excitation wavelength and the light-emission wavelength are in the visible light wavelength or higher, degradation of biological tissue and labeling material can be reduced due to irradiating with light. Furthermore, light scattering of a sample surface can be reduced, and the observation sensitivity can be increased. Note here that in an application using the light-emitting nanoparticles, when it is not necessary to consider damage to biological tissue and labeling material, the excitation wavelength and the light-emission wavelength are not necessarily in the visible light region.

Use of the light-emitting nanoparticles permits detection of cells, and treatment of animals. Specifically, the cell detection method of the present invention includes introducing the light-emitting nanoparticle into a cell, irradiating the light-emitting nanoparticle with light, and observing the cell. According to this detection method, because the light-emitting nanoparticle of the present embodiment has high fluorescence intensity and high sensitivity, observation of cells can be observed more easily.

The treatment method of an animal, described in the present specification without being claimed, is a method for treating a non-human animal. The method includes steps of administering the light-emitting nanoparticle to a non-human animal, irradiating light onto the light-emitting nanoparticle, and treating the animal. According to this treatment method, the light-emitting nanoparticle of the present embodiment has high fluorescence intensity and high sensitivity, and further, high biocompatibility, a condition of disease in an animal body can be detected with high sensitivity, and safely. Thus, it becomes possible to suitably treat a disease of a non-human animal.

Furthermore, the medical device of the present invention includes a testing portion for carrying out testing of an internal cell, a diagnosis portion for carrying out diagnosis of the internal cell, and/or a treatment portion for carrying out treatment of the internal cell, and further include a light irradiation portion which introduces the light-emitting nanoparticles into the internal cell, and irradiates the light-emitting nanoparticles with light in carrying out the testing, diagnosis, and/or treatment. Herein, examples of the testing portion for carrying out testing of an internal cell include a fluorescence endoscope for carrying out precision image diagnosis. Furthermore, examples of the diagnosis portion for carrying out diagnosing of an internal cell include a device for carrying out tissue biopsies. Furthermore, examples of the treatment portion for carrying out treatment of an internal cell include a device for extracting a tumor site by endoscopy. Furthermore, examples of the internal cell include cancer cells pertaining to oral cavity cancer, pharyngeal cancer, esophageal cancer, large intestinal cancer, small intestinal cancer, lung cancer, breast cancer, and bladder cancer. According to this medical device, the light-emitting nanoparticle of the present embodiment have high fluorescence intensity and high sensitivity, and high biocompatibility, whereby it becomes possible to carry out testing, diagnosis, and treatment of an internal cell with high sensitivity, and safely.

<Other applications>

Furthermore, the silicate-based base material and the rare-earth compound of the present invention can be used for applications other than the bioimaging application, and, for example, applications of light emitting devices such as a light emitting diode are expected.

EXAMPLES

Hereinafter, the present invention will be described with reference to Examples in more detail, but the present invention is not necessarily limited by these examples.

(Production of silicate-based base material 1)

Silicon tetrachloride as a raw material was subjected to hydrolysis reaction sufficiently in an oxygen-hydrogen flame to produce silica glass as porous synthetic quartz glass (soot body). At this time, particles that did not become porous synthetic quartz glass after hydrolysis in an oxygen-hydrogen flame and desorbed from the flame were used as the silicate-based base material 1. Note here that silicon tetrachloride was introduced from the center portion of the oxygen-hydrogen flame burner, and the length of the flame temperature zone of 1000°C or more was 400 mm, and the volume ratio ($H_2/O_2$) of hydrogen to oxygen was 1.5.

(Production of silicate-based base material 2)

Ultrapure water (22.8 mL), hexadecylammonium chloride (0.10 g), and 2N-sodium hydroxide aqueous solution (0.35 mL) were added to a screw tube, then 50 mg of silicate-based base material 1 was added thereto, and the obtained product was subjected to dispersion treatment by carrying out perform ultrasonic irradiation for 5 minutes. Tetraethoxysilane (0.55 mL) was then added and stirred at 1500 rpm for 48 hours at room temperature. Then, centrifugation (3000 rpm, 20 min) was carried out, washing was carried out twice with ultrapure water, dried at 120°C for two hours, and baked at 550°C for six hours. Thereafter, the obtained product was washed with 40 mL of ethanol, and subjected to solid-liquid separation by centrifugation, and the solid phase was dried at 120°C for two hours to obtain a silicate-based base material 2.

(Production of silicate-based base material 3)

A silicate-based base material 3 was obtained in the same manner as described above (production of the silicate-based base material 2) except that hexadecylammonium chloride and the silicate-based base material 1 were not added and the baking temperature was set at 750°C.

(Production of silicate-based base material 4)

A silicate-based base material 4 was obtained in the same manner as described above (production of the silicate-based base material 2) except that the silicate-based base material 1 was not added. Note here that all of the obtained silicate-based base materials 1 to 4 were spherical-shaped powders.

($Q_4/Q_3$ of silicate-based base material in solid $^{29}$Si-NMR spectrum)

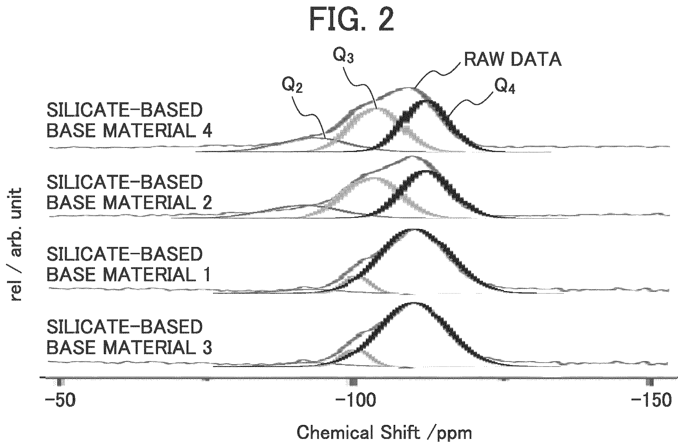

For the obtained silicate-based base materials 1 to 4, the solid $^{29}$Si-NMR spectrum was measured in the following conditions. The results are shown in FIG. 2.

<Measurement conditions>

Name of device: Bruker Advance 300wbs spectrometer (manufactured by BRUKER)
Measurement method: DD (Dipolar Decoupling) method
Sample tube: 7 mm
Number of revolution of sample: 5000 rpm
Resonance frequency: 59.62 MHz
Pulse width: 4.5 msec.
Waiting time: 60 sec
Multiplication time: 1000 times
Reference sample: hexamethylcyclotrisiloxane (-9.55 ppm)

<Peak separation method>

(Formation method of base line)

Operation (1): An average value 1 of strength in a range of - 70 to -79 ppm, and an average value 2 of strength in a range of -131 to -140 ppm are measured

Operation (2): A straight line passing through two points (-70 ppm, average value 1) and (-140 ppm, average value 2) is defined as a base line

Operation (3): The base line value is deleted from the spectrum

(Peak separation method)

Separation method: Solver function of Microsoft Office 2016 Excel (registered trademark)

Used function: Gaussian function (in the formula, A denotes a peak height, B denotes a peak position, and C denotes a half value width)

[Math. 1]

$$f(x) = A\exp\left\{-\frac{(x-B)^2}{C^2}\right\}$$

Peak assignment:

$Q_2$: -91 $\pm$ 2 ppm, two $\equiv$Si-O-Si$\equiv$ bonds and two $\equiv$Si-OH bonds
$Q_3$: -100 $\pm$ 2 ppm, three $\equiv$Si-O-Si$\equiv$ bonds and one $\equiv$Si-OH bond
$Q_4$: -110 $\pm$ 2 ppm, four $\equiv$Si-O-Si bonds

Initial conditions of A, B, and C:

$Q_2$: A=100000, B=-91, C=4
$Q_3$: A=400000, B=-100, C=5.5
$Q_4$: A=960000, B=-110, C=5.5

From FIG. 2, for each silicate-based base material, the obtained spectrum was defined as raw data, and the raw data were subjected to spectrum separation as mentioned above. $Q_4$ / $Q_3$ obtained when a peak area derived from $Si(OSi)_4$ was defined as $Q_4$ and the peak area derived from HO-Si(OSi)$_3$ was defined as $Q_3$ in the solid-state $^{29}$Si-NMR spectrum was 2.4 for the silicate-based base material 1, 1.6 for the silicate-based base material 2, 4.0 for the silicate-based base material 3, and 1.3 for the silicate-based base material 4. Not here that $Q_2$ shown in FIG. 2 is a peak derived from $(HO)_2$-Si(OSi)$_2$.

<Measurement of powder X-ray diffraction pattern of silicate-based base material>

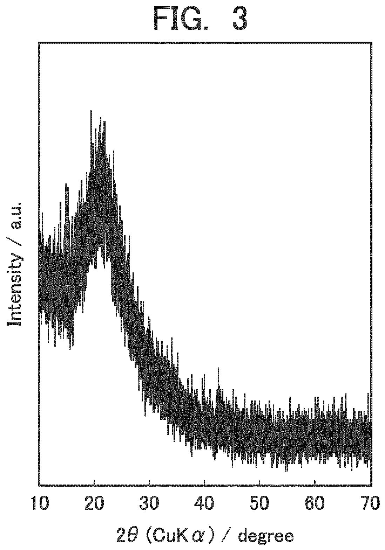

For the silicate-based base materials 1 to 4, the powder X-ray diffraction (XRD) patterns were measured. The measurement was carried out using a sample horizontal type X-ray diffractometer (XRD) (Smart Lab manufactured by Rigaku Corporation), under the conditions of X radiation source: CuK$\alpha$ radiation source ($\lambda$: 1.5418 Å), output: 40 kV/30 mA, scanning speed: 3.0°/min, sampling width: 0.01°, and measurement mode: continuous. As an example, the result of the silicate-based base material 1 is shown in FIG. 3. As a result, in the silicate-based base materials 1 to 4, the half value width of the amorphous halo pattern appearing in the vicinity of 2$\theta$ = 20° was large such as 6.7° to 8.3°, and a peak derived from crystal was not observed, showing an amorphous.

(Measurement of average particle size of silicate-based base material)

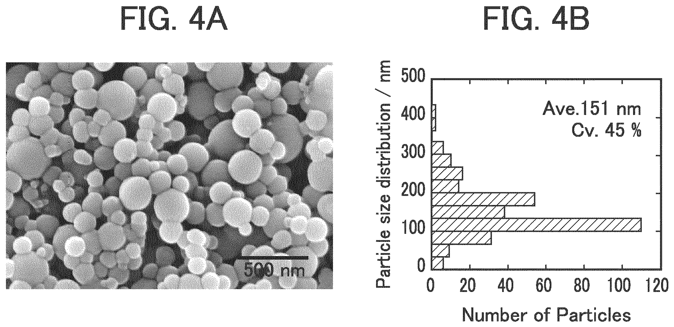

The silicate-based base materials 1 to 4 (particle powder) were fixed to a sample stand for observation with carbon paste, and dried. Next, particles were observed under a field emission scanning electron microscope (FE-SEM) (SU8230 manufactured by Hitachi High-Technologies, Ltd.), 100 or more of particle diameters were measured, and the average particle size was calculated. Specifically, for 300 particles, the longer diameter and the shorter diameter of each particle were measured, and "(longer diameter + shorter diameter) / 2" was defined as a particle diameter (particle size) of each particle. An average value of the particle diameter of each particle (a value obtained by dividing the total values of the particle diameter of each particle by number of particles (300)) was defined as an average particle size (Ave.), and coefficient of variance (Cv.) was calculated. As an example, the result of silicate-based base material 1 is shown in FIG. 4A and 4B. As a result, the silicate-based base material 1 had an average particle size of 151 nm, and the coefficient of variance of 45%. The silicate-based base material 2 had an average particle size of 124 nm, and the coefficient of variance of 52%. The silicate-based base material 3 had an average particle size of 143 nm, and the coefficient of variance of 44%. The silicate-based base material 4 had an average particle size of 129 nm, and the coefficient of variance of 52%.

(Example 1)

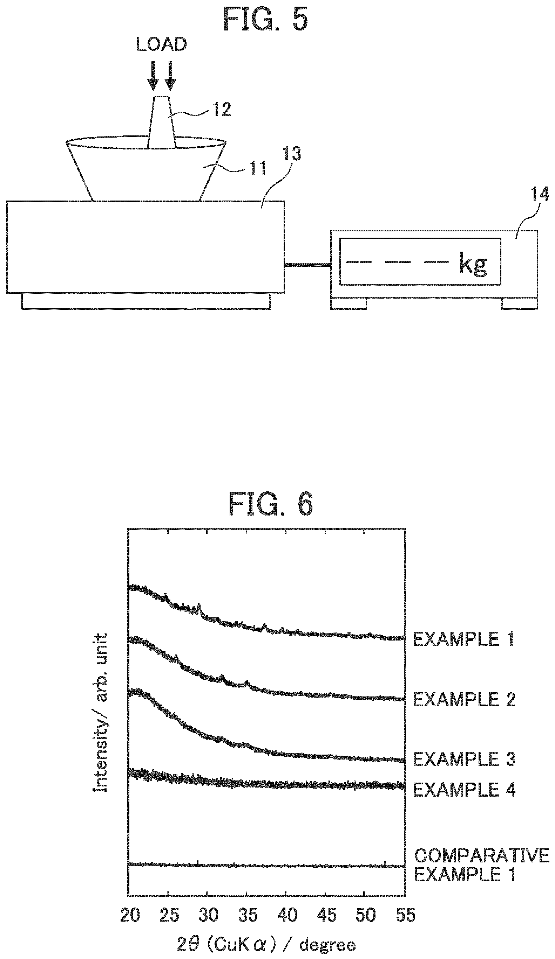

FIG. 5 is a schematic side view for illustrating a production device used in the production method of Example 1. As shown in FIG. 5, pulverization was carried out in an agate mortar 11 having a hemispherical recess space (diameter: 65 mm, and depth: 30 mm) and an 80-mm pestle 12 having a hemispherical protrusion (tip-end diameter: 20 mm, and height: 5 mm), the silicate-based base material and the europium chloride (III) hexahydrate were subjected to a solid-phase mechanochemical reaction under the load of 4 N. Specifically, europium chloride hexahydrate ($EuCl_3 \cdot 6H_2O$, manufactured by Wako Pure Chemical Industries, Ltd., special grade agent, purity: 99.9 wt.%) was added to 0.4 g (6.66 mmol) of the silicate-based base material 1 which had been dried at 120°C for 24 hours in the mortar 11, such that the percentage of the number of moles of Eu with respect to the total number of moles of Si and Eu (number of moles of Eu / (number of moles of Si + number of moles of Eu)) was 5.0 mol%. Then, for 5 minutes, the pestle 12 was revolved on the circumference of a radius of 25 mm in the recessed space in the mortar 11 by the pestle 12 without rotating the pestle 12 itself. The pestle 12 was revolved at 120 rotating/min with load of 4 N applied to the pestle 12. Note here that the above operation was carried out in a state in which the mortar 11 was mounted on an electronic balance 13, the load value was read by using the metric indicator 14 of the electronic balance 13. The obtained powder was dried at 120°C for two hours, and then baked at 550°C for 6 hours. Thereafter, the obtained product was washed with 40 mL of ethanol, and subjected to solid-liquid separation by centrifugation, and the solid phase was dried at 120°C for two hours to obtain the particles (spherical-shaped powder) of Example 1.

(Example 2)

Particles of Example 2 were obtained by carrying out the same operation as in Example 1 except that europium chloride hexahydrate was added such that the percentage of the number of moles of Eu with respect to the total number of moles of Si and Eu was 2.5 mol%.

(Example 3)

Particles of Example 3 were obtained by carrying out the same operation as in Example 1 except that europium chloride hexahydrate was added such that the percentage of the number of moles of Eu with respect to the total number of moles of Si and Eu was 1.25 mol%.

(Example 4)

Particles of Example 4 were obtained by carrying out the same operation as in Example 1 except that the europium chloride hexahydrate was added such that the percentage of the number of moles of Eu with respect to the total number of moles of Si and Eu was 0.625 mol%.

(Example 5)

Particles of Example 5 were obtained by carrying out the same operation as in Example 1 except that the silicate-based base material 2 was used instead of the silicate-based base material 1.

(Comparative Example 1)

Europium chloride hexahydrate was used as particles for Comparative Example 1.

(Comparative Example 2)

Particles of Comparative Example 2 were obtained by carrying out the same operation as in Example 1 except that the silicate-based base material 3 was used instead of the silicate-based base material 1.

(Comparative Example 3)

Particles of Comparative Example 3 were obtained by carrying out the same operation as in Example 1 except that the silicate-based base material 4 was used instead of the silicate-based base material 1.

<Powder X-ray diffraction (XRD)>

Particles of Examples 1 to 5 and Comparative Examples 1 to 3 were subjected to measurement of powder X-ray diffraction (XRD) pattern in the following conditions. Measurement was carried out using the sample horizontal type X-ray diffractometer (XRD, Smart Lab manufactured by Rigaku Corporation) under conditions of X radiation source: CuK$\alpha$ radiation source ($\lambda$: 1.5418 Å), output: 40 kV/30 mA, scanning speed: 3.0°/min, sampling width: 0.01°, and measurement mode: continuous. The diffraction peak position, diffraction angle, and half-value width were obtained by software (manufactured by Rigaku Corporation, software name: PDXL) attached to the device. The diffraction peaks were detected by the automatic profiling process of PDXL, by the second-order differential method (a method of detecting a region in which the peak second-order differential is negative (convex upward) as a peak), by removing the background, removing the K$\alpha_2$ line, and smoothing in this order, and fitting with the B-spline function (the split pseudo-Voigt function). The threshold (cut value of the standard deviation) in detecting the diffraction peak was 3.0. The threshold means that the diffraction peak is not regarded as a diffraction peak when the intensity of the diffraction peak is 3.0 times or less of its error. Three points were selected in descending order of the diffraction peak intensity (the height of the diffraction peak) and used for identification of the crystal. Furthermore, the crystallinity was calculated by the above formula (7). The results are shown in Table 1 and FIG. 6. In Table 1, in the upper stage, "m $\pm$ n" indicating the diffraction angle 2$\theta$ in which each diffraction peak is located indicates that the peak top position (diffraction peak top position) of the diffraction peak is m (°), the start point of the diffraction peak is m - n (°), and the end point of the diffraction peak is m + n (°). Specifically, the diffraction peak top was defined as the maximum intensity (maximum height) between the start point and the end point of each diffraction peak when a baseline is drawn from the start point to the end point. The position of the diffraction peak top (diffraction angle) was defined as the diffraction peak top position. In Table 1, the parentheses in the lower row indicate the half value width of each diffraction peak. The half value width indicates the width between diffraction peak positions having an intensity (height) of 50% of the diffraction peak top within the range of "m - n"° to "m + n"°.

As shown in Table 1 and FIG. 6, Examples 2 to 3 have the powder X-ray diffraction pattern having a diffraction peak (first diffraction peak) in a diffraction angle (2$\theta$) range of 34.3° to 36.1° in which a half value width of the diffraction peak in a diffraction angle (2$\theta$) range of 34.3° to 36.1° was 1.8° or less, and included a crystal of the compounds represented by the chemical formula (1) or (2). Note here that x in the chemical formula (1) was 0.2 or more and 0.6 or less. Furthermore, Examples 2 to 3 have a diffraction peak in a diffraction angle (2$\theta$) range of 25.9° to 26.5° and in a diffraction angle (2$\theta$) range of 31.6° to 32.2° in which the half value width of the diffraction peaks both in a diffraction angle (2$\theta$) range of 25.9° to 26.5° and a diffraction angle (2$\theta$) range of 31.6° to 32.2° was 0.6° or less.

Furthermore, Example 1 included a crystal of a compound represented by the chemical formula (3) or (4) having a powder X-ray diffraction pattern including diffraction peaks (second diffraction peak and third diffraction peak) in the diffraction angle (2$\theta$) range of 28.6° to 29.6° and a diffraction angle (2$\theta$) range of 36.8° to 38.4° in which the half-value width of the diffraction peak in the diffraction angle (2$\theta$) range of 28.6° to 29.6° was 1.0° or less and the half-value width of the diffraction peak in the diffraction angle (2$\theta$) range of 36.8° to 38.4° was 1.6° or less. Furthermore, Example 1 had diffraction peaks in the diffraction angle (2$\theta$) range of 39.0° to 40.2° in which the half-value width of the diffraction peak in the diffraction angle (2$\theta$) range of 39.0° to 40.2° was 1.2° or less. Furthermore, as shown in Table 1 and FIG. 6, in the particles obtained in Examples 1 to 3, crystallinity was more than 0, and the crystal of the europium compound was formed on the surface, while the particles of Example 4 to 5 and Comparative Examples 1 to 3 were amorphous. Note here that in any of Examples 1 to 5, a europium compound was bonded to the surface of each silicate-based base material.

[Table 1]

| | | Example | | | | | Comparative Example | | |
|---|---|---|---|---|---|---|---|---|---|
| | | 1 | 2 | 3 | 4 | 5 | 1 | 2 | 3 |
| Diffraction angle (2θ) in which diffraction peak is located: degree | | 24.8 + 0.4 (0.50) | 26.2 ± 0.3 (0.34) | 26.2 ± 0.3 (0.34) | - | - | - | - | - |
| (Half value width: degree) | | 27.5 ± 0.3 (0.37) | 31.9 ± 0.3 (0.37) | 31.9 ± 0.3 (0.36) | | | | | |
| | | 28.3 ± 0.3 (0.38) | 35.2 ± 0.9 (1.06) | 35.2 ± 0.9 (1.09) | | | | | |
| | | 29.1 ± 0.5 (0.59) | 45.9 ± 0.5 (0.61) | 45.9 ± 0.5 (0.59) | | | | | |
| | | 31.6 ± 0.6 (0.73) | | | | | | | |
| | | 34.5 ± 0.4 (0.49) | | | | | | | |
| | | 37.6 ± 0.8 (0.95) | | | | | | | |
| | | 39.6 ± 0.6 (0.73) | | | | | | | |
| | | 41.4 ± 0.6 (0.72) | | | | | | | |
| | | 48.1 ± 0.4 (0.50) | | | | | | | |
| Crystallinity | | 0.41 | 0.12 | 0.11 | 0.000 | 0.000 | 0.000 | 0.000 | 0.000 |

<Light-emitting property>

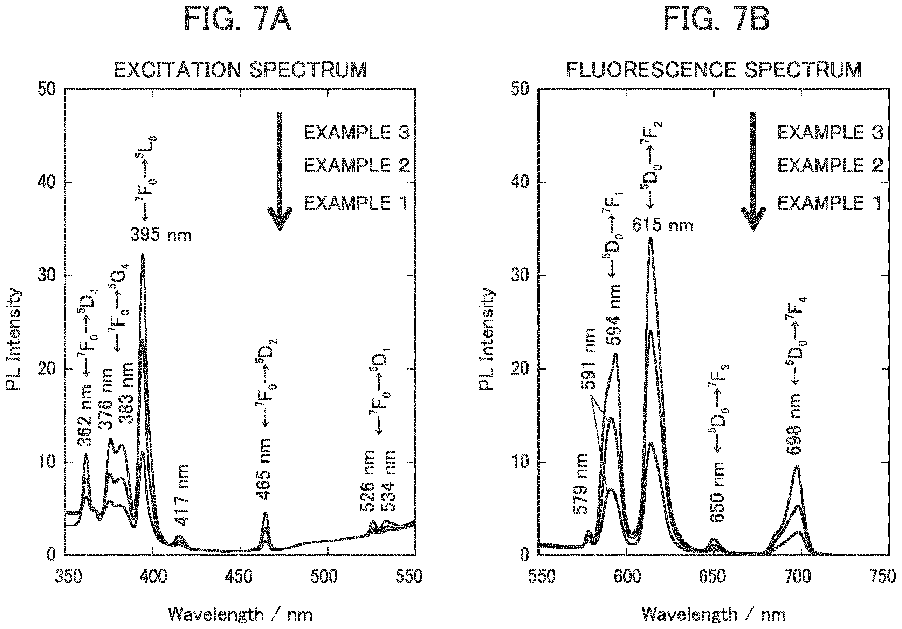

An excitation spectrum and a fluorescence spectrum of particles of Examples 1 to 5 and Comparative Examples 1 to 3 were measured in the following conditions. As an example, the results of Examples 1 to 3 are shown in FIG. 7A and 7B. FIG. 7A shows an excitation spectrum, and FIG. 7B shows a fluorescence spectrum.

- Excitation spectrum

The excitation spectrum was obtained by fixing the detection wavelength (615 nm) with a spectrophotometer (PL, FP-8500 manufactured by JASCO Corporation). The measurement conditions were atmosphere: air, excitation/detection slit size: 2.5 nm/2.5 nm, step width: 1.0 nm, sample weight: 20 mg, shape: pellet.

- Fluorescence spectrum

The sample was irradiated with excitation light from the Xe lamp using a spectrophotometer (PL, FP-8500 manufactured by JASCO Corporation) at room temperature (excitation wavelength: 395 nm), and a PL spectrum (fluorescence spectrum) was obtained. The measurement conditions were atmosphere: air, excitation/detection slit size:

2.5 nm / 2.5 nm, step width: 1.0 nm, sample weight: 20 mg, shape: pellet.

As a result, as shown in FIG. 7A and 7B, in all of Examples 1 to 5 and Comparative Examples 1 to 3, excitation peak derived from Eu(III) ion($^7F_0 \rightarrow ^5D_4$ transition, $^7F_0 \rightarrow ^5G_4$ transition, $^7F_0 \rightarrow ^5L_6$ transition, $^7F_0 \rightarrow ^5D_2$ transition, and $^7F_0 \rightarrow ^5D_1$ transition) and light-emitting peak ($^5D_0 \rightarrow ^7F_1$ transition, $^5D_0 \rightarrow ^7F_2$ transition, $^5D_0 \rightarrow ^7F_3$ transition, $^5D_0 \rightarrow ^7F_4$ transition) were observed. Furthermore, from FIG. 7A and 7B, the internal quantum yields of the particles of particles of Examples 1 to 5 and Comparative Examples 1 to 3 at the excitation wavelength $\lambda_{ex}$ = 395 nm and the emission wavelength $\lambda_{em}$ = 615 nm were respectively 1.0 to 4.0%.

<Chemical composition (X-ray fluorescence analysis (XRF))>

Particles of Examples 1 to 5 and Comparative Examples 1 to 3 were subjected to the X-ray fluorescence analysis (XRF) in the following conditions. A sample pellet obtained by pressurizing about 100 mg of sample powder which had not been diluted was subjected to XRF analysis using the fluorescent X-ray analyzer (XRF, manufactured by Rigaku Corporation, device name: ZSX PrimusII) and software attached to the device (manufactured by Rigaku Corporation, name of the software: EZ scan program). In measurement, without using a primary filter and a calibration curve, semi-quantitative analysis was carried out using the measurement program attached to the device is used. Analysis results and results converted in element ratios are shown in Tables 2 to 5.

[Table 2]

|  | Si (mol%) | O (mol%) | Eu (mol%) | C (mol%) | Cl (mol%) | Other element (mol%) |
|---|---|---|---|---|---|---|
| Example 1 | 30.7 | 64.1 | 1.57 | 2.67 | 0.959 | 0.001 |
| Example 2 | 32.0 | 64.4 | 0.844 | 2.22 | 0.494 | 0.042 |
| Example 3 | 32.0 | 65.0 | 0.404 | 2.41 | 0.239 | - |
| Example 4 | 32.1 | 63.5 | 0.195 | 2.01 | 0.045 | 2.15 |
| Comparative Example 1 | - | 25.6 | 21.5 | - | 52.9 | 0 |

[Table 3]

|  | Eu/Si (mol%) | Cl/Eu (mol%) | C/Si (mol%) | C/Eu (mol%) |
|---|---|---|---|---|
| Example 1 | 5.11 | 61.1 | 8.70 | 170 |
| Example 2 | 2.64 | 58.5 | 6.94 | 263 |
| Example 3 | 1.26 | 59.2 | 7.53 | 597 |
| Example 4 | 0.607 | 23.1 | 6.26 | 1031 |
| Comparative Example 1 | - | 246 | - | - |

[Table 4]

|  | Si (mol%) | O (mol%) | Eu (mol%) | C (mol%) | Cl (mol%) | N (mol%) | Other element (mol%) |
|---|---|---|---|---|---|---|---|
| Example 1 | 30.7 | 64.1 | 1.57 | 2.67 | 0.959 | - | 0.001 |
| Example 5 | 22.5 | 45.0 | 1.16 | 30.6 | 0.139 | 0.560 | 0.041 |
| Comparative Example 2 | 30.1 | 63.6 | 0.505 | 5.81 | 0.0216 | - | - |
| Comparative Example 3 | 20.4 | 45.2 | 1.52 | 32.5 | 0.0869 | 0.179 | 0.114 |

[Table 5]

|  | Eu/si (mol%) | Cl/Eu (mol%) | C/Si (mol%) | C/Eu (mol%) |
|---|---|---|---|---|
| Example 1 | 5.11 | 61.1 | 8.70 | 170 |
| Example 5 | 5.16 | 12.0 | 136 | 2638 |

(continued)

|  | Eu/si (mol%) | Cl/Eu (mol%) | C/Si (mol%) | C/Eu (mol%) |
|---|---|---|---|---|
| Comparative Example 2 | 1.68 | 4.28 | 19.3 | 1150 |
| Comparative Example 3 | 7.45 | 5.72 | 159 | 2138 |

<Transmission electron microscope (TEM) observation>

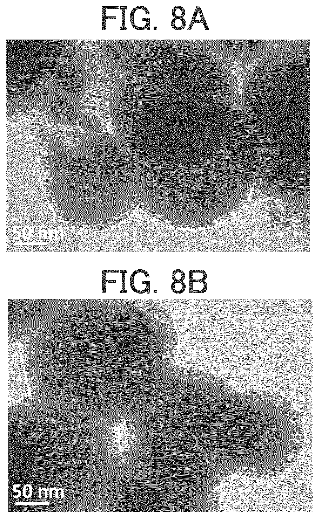

Particles of Examples 1 to 4 were observed under transmission electron microscope (TEM). Firstly, the particles were dispersed in ethanol at a concentration of 0.1 mass%, subjected to ultrasonication for 15 minutes, and dried on a carbon microgrid. Then, the center portion of the particle membrane was evaluated and analyzed under transmission electron microscopy (TEM) (HT 7700, manufactured by Hitachi High-Technologies, Ltd.). As an example of the results, the result of Example 1 is shown in FIG. 8A, and the result of Example 2 is shown in FIG. 8B. As a result, in Examples 1 and 2, a layer covering the surface of the silicate-based base material was observed, and the covering layer has a thickness of 19 to 25 nm in Example 1, and 8 to 12 nm in Example 2. Note here that in Examples 3 and 4, formation of a clear layer was not observed.

<Measurement of average particle size of particles>

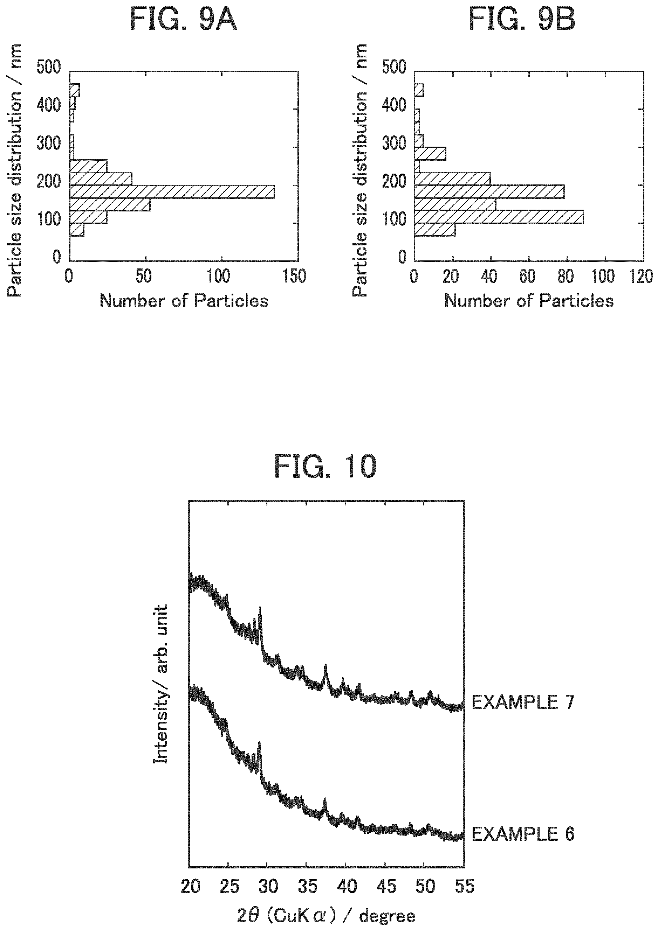

For the particles of Examples 1 to 4, 100 or more particle diameters were measured using the field emission-type scanning electron microscope (FE-SEM), and an average particle size was calculated. Note here that for 300 particles, the longer diameter and the shorter diameter of each particle were measured, respectively, and "(longer diameter + shorter diameter) / 2" was defined as a particle diameter of each particle. An average value of the particle diameters of each particle (a value obtained by dividing the total values of the particle diameters of each particle by the number of particles (300)) was defined as an average particle size (Ave.). As an example, Example 1 is shown in FIG. 9A, and Example 2 is shown in FIG. 9B. As a result, the average particle size of the particles of Example 1 was 189 nm, and the thickness of the silicate-based base material 1 was 151 nm, from these values, the thickness of the layer was calculated to be (189-151)/2 of 19 nm. The average particle size of the particles of Example 2 was 172 nm, and the thickness of the silicate-based base material 1 was 151 nm, from these values, the thickness of the layer was calculated to be (172-151)/2 of 10 nm. An average particle size of the particles of Example 3 was 160 nm. An average particle size of the particles of Example 4 was 155 nm.

(Example 6)

Particles of Example 6 were obtained by the same operation as in Example 1 except that the load was made to be 8.0 N.

(Example 7)

Particles of Example 7 were obtained by the same operation as in Example 1 except that the load was made to be 12.0 N.

For the particles of Examples 6 to 7, results obtained in the same manner as in <Powder X-ray diffraction (XRD)> mentioned above are shown in Table 6 and FIG. 10. As shown in Table 6 and FIG. 10, both Examples 6 to 7 included a crystal of the compound represented by the chemical formula (3) or (4) having diffraction peaks (a second diffraction peak and a third diffraction peak) in a diffraction angle (2θ) range of 28.6° to 29.6° and the diffraction angle (2θ) range of 36.8° to 38.4°, in which a half value width of the diffraction peak in the range of the diffraction angle (2θ) range of 28.6° to 29.6° was 1.0° or less, a half value width of the diffraction peak in the range of the diffraction angle (2θ) range of 36.8° to 38.4° was 1.6° or less. Furthermore, both Examples 6 to 7 had a diffraction peak in a diffraction angle (2θ) range of 39.0° to 40.2° in which a half value width in a diffraction angle (2θ) range of 39.0° to 40.2° was 0.8° or less. As shown in Table 6 and FIG. 10, particles obtained in both of Examples 6 to 7 had crystallinity of more than 0, and had a crystal of a europium compound on the surface of the particle. As shown in Table 6 and FIG. 10, it is found that as the load is increased, the crystallinity can be enhanced.

[Table 6]

|  | Example 6 | Example 7 |
|---|---|---|
| Diffraction angle (2θ) in which diffraction peak is located : degree | 24.8 ± 0.6 (0.70) | 24.8 ± 0.5 (0.61) |
|  | 27.0 ± 0.1 (0.14) | 27.0 ± 0.1 (0.13) |
|  | 27.5 ± 0.3 (0.31) | 27.5 ± 0.3 (0.37) |
| (Half value width : degree) | 28.3 ± 0.3 (0.30) | 28.3 ± 0.3 (0.36) |
|  | 29.1 ± 0.5 (0.54) | 29.1 ± 0.5 (0.54) |
|  | 31.3 ± 0.3 (0.41) | 31.3 ± 0.3 (0.41) |
|  | 34.5 ± 0.5 (0.64) | 34.5 ± 0.3 (0.38) |
|  | 37.4 ± 0.6 (0.73) | 37.4 ± 0.7 (0.84) |
|  | 39.4 ± 0.5 (0.60) | 39.4 ± 0.6 (0.72) |
|  | 41.6 ± 0.5 (0.58) | 41.6 ± 0.6 (0.72) |
|  | 46.4 ± 0.7 (0.79) | 46.4 ± 0.8 (0.91) |
|  | 48.2 ± 0.5 (0.54) | 48.2 ± 0.5 (0.54) |
|  | 50.6 ± 0.3 (0.40) | 50.6 ± 0.4 (0.48) |
|  | 51.8 ± 0.5 (0.64) | 51.8 ± 0.4 (0.52) |
| Crystallinity | 0.51 | 0.52 |

<Toxicity evaluation>

Particles of Examples 1 to 4 or Comparative Example 1 were subjected to toxicity evaluation by cancer cell imaging and a fluorescence intensity measurement by the following methods.

(Modification of cancer cell binding molecule (folic acid derivative FA-NHS) to particle)

To 250 mg of the particles of Examples 1 to 4 or Comparative Example 1, 12 ml of an HCl aqueous solution (pH = 2) was added, and ultrasonic treatment was carried out. Next, a solution containing 0.78 ml (3.3 mmol) of 3-aminopropyltriethoxysilane (APTES) in 5 mL of ethanol was prepared, and added to the ultrasonic-treated solution to obtain a mixed solution. The mixed solution was stirred for 20 hours at 40°C (pH < 6.5). After stirring was completed, the mixed solution was centrifuged and washed with ethanol. After washing, drying was carried out under reduced pressure to obtain 150 mg of particles having a surface modified with APTES. To 150 mg of these particles having a surface modified with APTES, 25 mL of a 50 mM phosphate buffer solution (pH = 7.0) was added, and ultrasonic treatment was carried out. Next, a solution containing 430 mg (0.8 mmol) of FA-NHS (folic acid derivative) in 12 mL of dimethylsulfoxide (DMSO) was prepared, and the prepared solution was added to the ultrasonic-treated solution to obtain a mixed solution. The mixed solution was stirred for 3 hours at room temperature. After stirring was completed, the mixed solution was centrifuged and washed with water. After washing, drying was carried out under reduced pressure to obtain FA (folic acid)-modified particles of Examples 1 to 4 or Comparative Example 1.

(Cell culture)

Hela cancer cells were cultured in a PS flask (dissemination density: 100 x $10^4$ cells/37 $cm^2$). Thawing and dissemination were carried out for 7 days. The cells were peeled off and separated. The Hela concentration was (0.99 ± 0.07) x $10^5$ cells/mL. Concentration of the cells was adjusted, and 10 vol% FBS (fetal bovine serum) was cultured in DMEM (Dulbecco modification nutrient medium). The obtained cells were 7.5 x $10^4$ cells per 1 mL. An amount of 2.25 mL/TCPS was disseminated to polystyrene dishes (TCPS) (cultivation area: 9.6 $cm^2$), and the dissemination density was 1.8 x $10^4$ cells/$cm^2$. (Microscope observation). Thereafter, culturing was carried out (temperature: 37°C, $CO_2$ concentration: 5%, humidity: 100%). After 12 hours, FA (folic acid)-modified particles of Examples 1 to 4 or Comparative Example 1 were added to 10 vol% DMEM, and dispersed, and the concentration was adjusted to 100 mg/mL.
For living cell imaging, at 3 hours, 12 hours, 24 hours, 36 hours and 48 hours after spraying FA (folic acid)-modified particles on the cell surface, the medium was removed.

(Measurement of cell density)

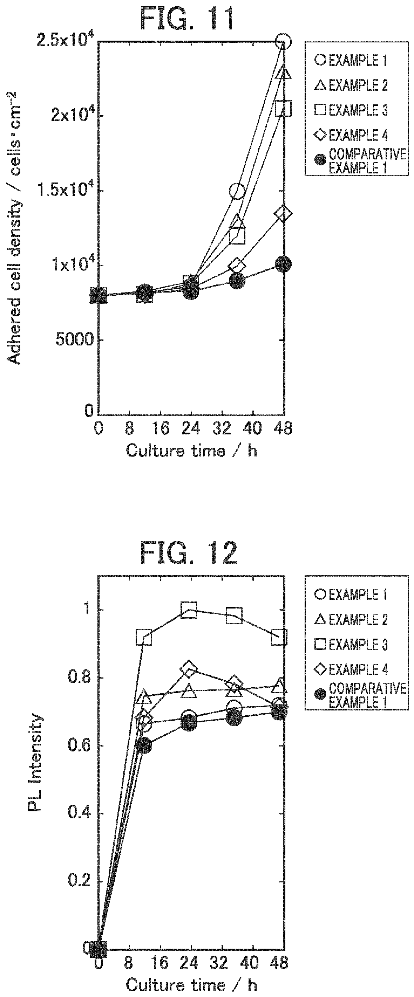

After the above cells were cultured, a polystyrene dish (TCPS) including cells was washed twice with 1 ml of phosphate buffered saline (PBS), then FA (folic acid)-modified particles which had not taken up by the cells were removed, 0.1 ml of 0.05% Trypsin-EDTA was placed in a TCPS including cells, followed by allowing to stand still in a $CO_2$ incubator for 12 minutes, and cells were peeled off from TCPS. Peeling was observed, a suspension including cells was taken into a 50-ml conical tube and subjected to centrifugation (2000 rpm, 2 min). After centrifugation, a supernatant was discarded, 7 ml of culture medium was added to the tube, overturning stirring was carried out about 10 times, and then centrifugation (2000 rpm, 2 min) was carried out. Thereafter, the supernatant was discarded, 20 ml of medium was added to the tube, pipetting was carried out about 20 times, 1 ml was aliquoted into 15 ml tubes, the cell suspension was placed into a disposable hemocytometer using a micropipette outside a clean bench, the cell number was confirmed under a microscope, and the average value (cell/cm$^2$) was calculated. At 3 hours, 12 hours, 24 hours, 36 hours, and 48 hours after spraying FA (folic acid)-modified particles on the cell surface, the average value of cell density was calculated, and the results are shown in FIG. 11.

(Measurement of fluorescence intensity (PL))

Using an integrating sphere (ISF834 manufactured by JASCO Corporation), PL spectrum measurement was carried out by a spectrophotometer (PL; FP-8500 manufactured by JASCO Corporation), under the conditions: atmosphere: air, excitation/detection slit size: 10 nm/10 nm, a step width: 1.0 nm. Specifically, TCPS including cells after cell culture and FA (folic acid)-modified particles was washed with 1 ml of phosphate buffered saline twice, FA (folic acid)-modified particles which had not been taken up by the cells were removed, and further washing with 1 ml of ultrapure water twice. TCPS after washing (i.e., TCPS including cells which had taken up FA (folic acid)-modified particles) were freezedried. After drying, the cell layer present in the TCPS was peeled off to form a powder, and the powder was placed into a powder cell holder. Then, the PL spectrum was measured. The excitation wavelength herein was 395 nm, and integrated light-emission intensity centered on the peak top of $^5D_0 \rightarrow ^7F_2$ transition was calculated. Specifically, the PL spectrum area integrated light-emission intensity in the wavelength region between 600 to 635 nm was obtained. Note here that light emission from TCPS was not observed in this wavelength region. The results are shown in FIG. 12.

As shown in FIG. 11, Examples 1 to 3 in which crystal of a europium compound were formed on the surface, exhibited a normal cell proliferation behavior and no cytotoxicity. In other words, it can be said that, in a case of compounding (compositing) into a spherical silicate-based base material as a crystal, no cytotoxicity was observed. On the other hand, in Comparative Example 1, a normal cell proliferation behavior is not observed, and it is considered that since the amorphous $EuCl_3 \cdot 6H_2O$ elemental substance is an unstable chloride, it eluted into the cell culture solution to form Eu ion or Eu chloride ion, and reacted directly with the cells. Similarly, also when the amorphous of Example 4 were used, it is considered that the amorphous particles were eluted into the cell culture solution to form Eu ions or Eu chloride ions and reacted directly with the cells. Therefore, in the case of an amorphous, it can be said that there is cytotoxicity due to the elution.

As shown in FIG. 12, for the fluorescence property, in Comparative Example 1 showing a non-crystal, the fluorescence intensity associated with binding and uptake into cells by elution and toxicity was low in culture time of 48 hours, but in Examples 1 to 3 showing crystal, the fluorescence intensity in culture time of 48 hours was higher than in Comparative Example 1 because there was no elution and no toxicity.

(Measurement of fluorescence integrated intensity)

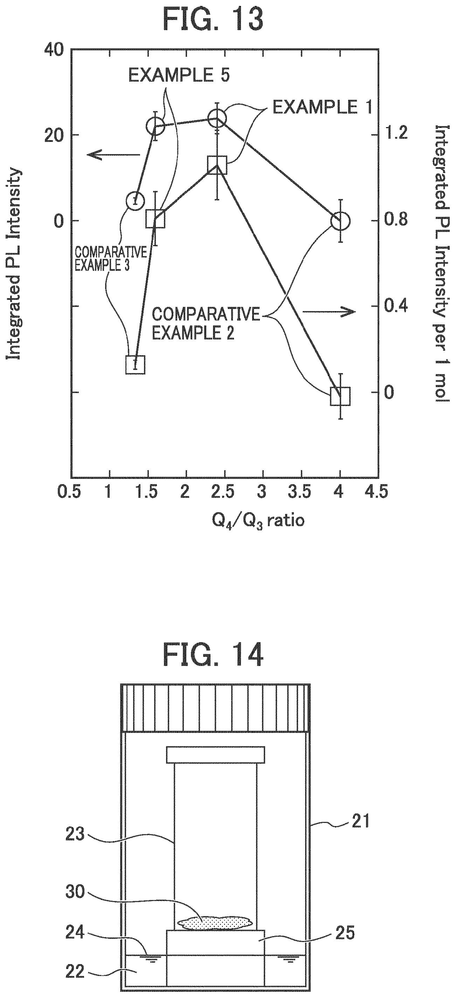

PL spectra of particles of Examples 1 and 5 and Comparative Examples 2 and 3 were measured. A PL spectrum (fluorescence spectrum) was obtained by irradiating a sample with excitation light (excitation wavelength: 394 nm) from the Xe lamp using a spectrophotometer (PL, FP-8500 manufactured by JASCO Corporation) at room temperature under the conditions: atmosphere: air, excitation/detection slit size: 2.5 nm / 2.5 nm, a step width: 1.0 nm, sample mass: 20 mg, shape: pellet. Thereafter, the integrated light-emission intensity centered on the peak top of the $^5D_0 \rightarrow ^7F_2$ transition (left vertical axis in FIG. 13) was calculated. Specifically, the calculation was carried out using the PL spectrum area in the wavelength region between 600 nm to 635 nm. Furthermore, from the results of XRF, the amount of $Eu^{3+}$ in particles (in 20 mg each) was obtained, and integrated light-emission intensity per mole (right vertical axis in FIG. 13) was obtained. For the integrated emission intensity per mole, the highest integrated emission intensity was fixed at 1 and the relative intensities of the other samples were calculated. The results are shown in FIG. 13.

As shown in FIG. 13, Examples 1 and 5 in which $Q_4/Q_3$ was 1.6 to 3.9 showed high fluorescence intensity. In more detail, Example 1 in which $Q_4/Q_3$ was 2.4 had especially high fluorescence intensity. This is because the support amount is optimized, Eu(III) ions are present in monodisperse on the surface of the silicate-based base material (silica sphere). In Example 5 in which $Q_4/Q_3$ was 1.6, the fluorescence intensity is high but lower than that of Example 1. This is because the amount of silanol group in the silicate-based base material (silica sphere) was larger than in Example 1 and Eu(III) ions agglomerated more than in Example 1. On the other hand, in Comparative Examples 2 and 3 in which $Q_4/Q_3$ was out of a range of 1.6 to 3.9, the fluorescence intensity was low. In more detail, in Comparative Example 3 in which $Q_4/Q_3$ was 1.3, the fluorescence intensity was low. This is because the amount of silanol group in the silicate-based base material was large, the support amount of the Eu compound became too large, and Eu(III) ions agglomerated. In Comparative Example 2 in which $Q_4/Q_3$ was 4.0, the fluorescence intensity was low. This is because the amount of the silanol group in the silicate-based base material is small, and the support amount of Eu(III) ions is small.

(Example 8)

As shown in FIG. 14 being a schematic side view for illustrating a production method of Example 8, into a hermetically-sealable cylindrical-shaped container 21 made of PFA (tetrafluoroethylene-perfluoroalkylvinyl ether copolymer) having a volume of 30 mL, 2.5 mL of ammonia water was placed, and a stand 25 was mounted in the container 21 so that the 10 mL-screw tube 23 was placed to be higher than a liquid surface 24 of ammonia water 22. A mixed and pulverized product 30 was obtained by mixing and pulverizing 10 mg of a silicate-based base material (silicate-based base material 1) and 10 mg of europium chloride (III) hexahydrate. The obtained mixed and pulverized product 30 was placed in a 10-mL screw tube 23, the screw tube 23 was mounted on the stand 25 of the container 21 with the upper portion opened without being lidded, and the container 21 was sealed and allowed to stand still at 60°C for 24 hours. Thus, the inside of the container 21 was saturated with ammonia vapor, and the surface of the silicate-based base material was dissolved by deliquescence and ammonia vapor. Thereafter, the screw tube 23 was taken out form the container 21. Thus, the dissolved silicate-based base material was deposited again and coprecipitated with Eu$^{3+}$ on the surface of the silicate-based base material, and thereby, a rare-earth compound and NH$_4$Cl as a crystal were deposited on the surface of the silicate-based base material. The obtained powder was dried at 120°C for 2 hours, and baked at 550°C for 6 hours. Thereafter, the obtained product was washed with 40 mL of ethanol, solid-liquid separated by centrifugation, and the solid phase was dried at 120°C for two hours to obtain spherical-shaped powder of Example 8.

(Example 9)

A mixed dispersion was obtained by mixing 10 mg of silicate-based base material (silicate-based base material 1), and 1.0 mL of europium chloride (III) aqueous solution having a concentration of 10 g/L, followed by ultrasonication for 2 minutes. Particles of Example 9 were obtained by carrying out the same operation as in Example 8 except that 0.1 mL of the obtained mixed dispersion was used instead of the mixed and pulverized product.

(Example 10)

Particles of Example 10 were obtained by carrying out the same operation as in Example 9 except that the amount of mixed dispersion was made to be 1.0 mL.

<Powder X-ray diffraction (XRD)>

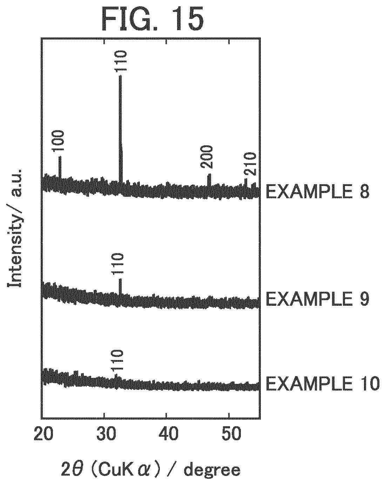

For particles of Examples 8 to 10, results obtained in the same manner as in the above <Powder X-ray diffraction (XRD)> are shown in Table 7 and FIG. 15.
As shown in Table 7 and FIG. 15, in Examples 8 to 10, a crystal of NH$_4$Cl was formed on the surface of the silicate-based base material. Note here that in all of Examples 8 to 10, a europium compound was bonded to the surface of the silicate-based base material.

[Table 7]

| | Example 8 | Example 9 | Example 10 |
|---|---|---|---|
| Diffraction angle (2θ) in which diffraction peak is located : degree | 22.9 ± 0.01 (0.013) | 32.6 ± 0.02 (0.025) | 32.6 ± 0.02 (0.025) |
| | 32.6 ± 0.02 (0.025) | | |
| | 40.3 ± 0.05 (0.060) | | |
| (Half value width : degree) | 46.8 ± 0.03 (0.038) | | |
| | 52.8 ± 0.05 (0.060) | | |
| | 54.1 ± 0.04 (0.050) | | |

(continued)

|  | Example 8 | Example 9 | Example 10 |
|---|---|---|---|
| Crystallinity | 0.060 | 0.012 | 0.0039 |

<Chemical composition (X-ray fluorescence analysis (XRF))>

For particles of Examples 8 to 10, an X-ray fluorescence analysis (XRF) was carried out as in Example 1. Results are shown in Tables 8 and 9.

[Table 8]

|  | Si (mol%) | O (mol%) | Eu (mol%) | C (mol%) | Cl (mol%) | N (mol%) | Other element (mol%) |
|---|---|---|---|---|---|---|---|
| Example 8 | 22.2 | 65.4 | 1.02 | 6.28 | 4.96 | 0.0638 | 0.0762 |
| Example 9 | 27.2 | 66.2 | 0.179 | 5.54 | 0.868 | 0.0527 | - |
| Example 10 | 23.8 | 69.6 | 0.034 | 6.70 | 0.152 | 0.0302 | - |

[Table 9]

|  | Eu/Si (mol%) | Cl/Eu (mol%) | C/Si (mol%) | C/Eu (mol%) |
|---|---|---|---|---|
| Example 8 | 4.59 | 486 | 28.3 | 616 |
| Example 9 | 0.658 | 485 | 20.4 | 3095 |
| Example 10 | 0.143 | 447 | 28.2 | 19706 |

(Example 11)

Particles of Example 11 were produced by using a production device of FIG. 5. As shown in FIG. 5, pulverization was carried out in an agate mortar 11 having a hemispherical recess space (diameter: 65 mm, and depth: 30 mm) and an 80-mm pestle 12 having a hemispherical protrusion (tip-end diameter: 20 mm, and height: 5 mm), the silicate-based base material 1 and europium fluoride were subjected to a solid-phase mechanochemical reaction under the load of 4 N. Specifically, europium fluoride ($EuF_3$, manufactured by Wako Pure Chemical Industries, Ltd., special grade agent, purity: 99.9 wt.%) was added to 0.4 g (6.66 mmol) of the silicate-based base material 1 which had been dried at 120°C for 24 hours in the mortar 11 such that the percentage of the number of moles of Eu with respect to the total number of moles of Si and Eu was 1.3 mol%. Then, for 5 minutes, the pestle 12 was revolved on the circumference of a radius of 25 mm in the recessed space in the mortar 1 by the pestle 12 without rotating the pestle 12 itself. The pestle 12 was revolved at 120 rotating/min with load of 4 N applied to the pestle 12. Note here that the above operation was carried out in a state in which the mortar 11 was mounted on an electronic balance 13, the load value was read by using the metric indicator 14 of the electronic balance 13. The obtained powder was dried at 120°C for two hours, and then baked at 550°C for 6 hours. Thereafter, the obtained product was washed with 40 mL of ethanol, and subjected to solid-liquid separation by centrifugation, and the solid phase was dried at 120°C for two hours to obtain the particles (spherical-shaped powder) of Example 11.

(Example 12)

Particles of Example 12 were obtained by carrying out the same operation as in Example 11 except that europium fluoride was added such that the percentage of the number of moles of Eu with respect to the total number of moles of Si and Eu was 2.5 mol%.

(Example 13)

Particles of Example 13 were obtained by carrying out the same operation as in Example 11 except that europium fluoride was added such that the percentage of the number of moles of Eu with respect to the total number of moles of Si and Eu was 5.0 mol%.

(Comparative Example 4)

Europium fluoride was used as a particle of Comparative Example 4.

<Powder X-ray diffraction (XRD)>

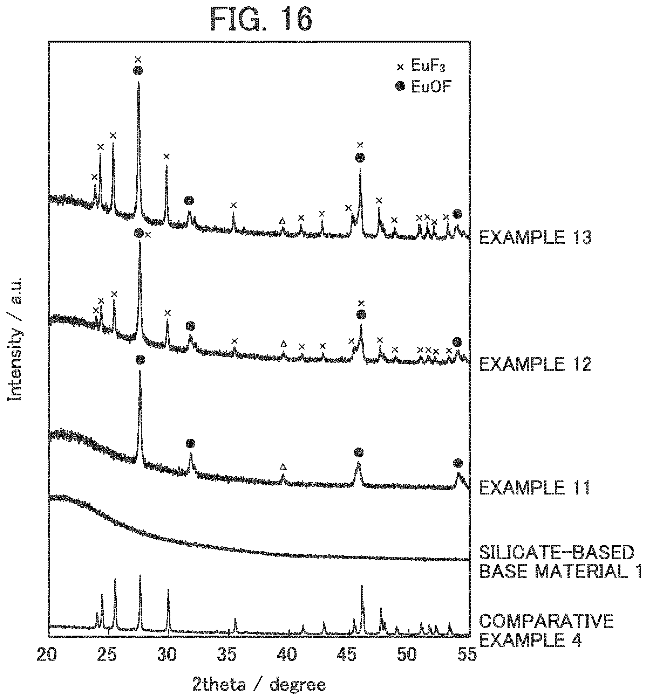

For the particles of Examples 11 to 13 and Comparative Example 4, results obtained in the same manner as in the <Powder X-ray diffraction (XRD)> mentioned above are shown in Table 10 and FIG. 16. FIG. 16 also shows the powder X-ray diffraction pattern of only silicate-based base material 1.

As shown in Table 10 and FIG. 16, Examples 12 and 13 included a crystal of a compound represented by the formula (5) having a powder X-ray diffraction pattern having a diffraction peak (fourth diffraction peak) in a diffraction angle $(2\theta)$ range of 26.6° to 28.6°, a diffraction peak (fifth diffraction peak) in a diffraction angle $(2\theta)$ range of 44.8° to 46.8°, and a diffraction peak (seventh diffraction peak) in a diffraction angle $(2\theta)$ range of 30.8° to 32.8°, respectively, in which a half value width of the fourth diffraction peak was 0.3° or less, a half value width of the fifth diffraction peak was 0.57° or less, and a half value width of the seventh diffraction peak was 0.38° or less, and a crystal of a compound represented by the formula (6) having a powder X-ray diffraction pattern having a diffraction peak (fourth diffraction peak) in a diffraction angle $(2\theta)$ range was 26.6° to 28.6°, a diffraction peak (fifth diffraction peak) in a diffraction angle $(2\theta)$ range was 44.8° to 46.8°, and a diffraction angle $(2\theta)$ was 24.3 to 26.3 (sixth diffraction peak), respectively, in which a half value width of the fourth diffraction peak was 0.3° or less, a half value width of the fifth diffraction peak was 0.57° or less, and a half value width of the sixth diffraction peak was 0.22° or less. Example 11 is a crystalline monophase of a compound represented by the formula (5) having a powder X-ray diffraction pattern having a diffraction peak (fourth diffraction peak) in a diffraction angle $(2\theta)$ range of 26.6° to 28.6°, a diffraction peak (fifth diffraction peak) in a diffraction angle $(2\theta)$ range of 44.8° to 46.8°, and a diffraction peak (seventh diffraction peak) in a diffraction angle $(2\theta)$ range of 30.8° to 32.8°, respectively, in which a half value width of the fourth diffraction peak was 0.3° or less, a half value width of the fifth diffraction peak was 0.57° or less, and a half value width of the seventh diffraction peak was 0.38° or less. The particles of Examples 12 to 13 are said to be a mixed crystal of the compound represented by the formula (5), the compound represented by the formula (6), and an amorphous silica. Furthermore, in Examples 11 to 13, the diffraction peak $(2\theta)$ were observed in 39.1° to 39.7°. In FIG. 16, they are described as "Δ", and used in calculation of the crystallinity of Table 10.

[Table 10]

| | | Example 11 | Example 12 | Example 13 | Comparative Example 4 |
|---|---|---|---|---|---|
| Diffraction angle $(2\theta)$ in which diffraction peak is located: degree<br><br>(Half value width: degree) | | 27.6 ± 0.1 (0.11) | 24.3 ± 0.05 (0.060) | 24.3 ± 0.03 (0.033) | 24.3 ± 0.04 (0.048) |
| | | 28.0 ± 0.1 (0.12) | 25.4 ± 0.05 (0.066) | 25.4 ± 0.05 (0.054) | 25.4 ± 0.05 (0.054) |
| | | 32.4 ± 0.06 (0.072) | 27.5 ± 0.09 (0.11) | 27.5 ± 0.07 (0.084) | 27.5 ± 0.04 (0.048) |
| | | 46.1 ± 0.1 (0.12) | 29.8 ± 0.04 (0.048) | 29.8 ± 0.04 (0.045) | 29.8 ± 0.04 (0.047) |
| | | 46.7 ± 0.1 (0.11) | 31.7 ± 0.1 (0.12) | 31.7 ± 0.13 (0.16) | 35.4 ± 0.04 (0.047) |
| | | 54.7 ± 0.1 (0.12) | 35.4 ± 0.04 (0.048) | 35.4 ± 0.04 (0.045) | 41.0 ± 0.04 (0.048) |
| | | | 41.0 ± 0.04 (0.048) | 41.0 ± 0.04 (0.045) | 42.8 ± 0.04 (0.048) |
| | | | 42.8 ± 0.04 (0.046) | 42.8 ± 0.05 (0.057) | 45.5 ± 0.05 (0.054) |
| | | | 45.5 ± 0.3 (0.36) | 45.5 ± 0.3 (0.33) | 47.6 ± 0.05 (0.054) |
| | | | 47.6 ± 0.07 (0.084) | 47.6 ± 0.05 (0.053) | 51.6 ± 0.04 (0.048) |
| | | | 51.6 ± 0.05 (0.066) | 51.6 ± 0.05 (0.054) | 52.1 ± 0.05 (0.057) |
| | | | 53.3 ± 0.05 (0.060) | 53.3 ± 0.05 (0.054) | 53.3 ± 0.04 (0.048) |
| | | | 54.5 ± 0.1 (0.13) | 54.5 ± 0.2 (0.21) | |
| Crystallinity | | 0.076 | 0.11 | 0.12 | 0.044 |

<Light-emitting property>

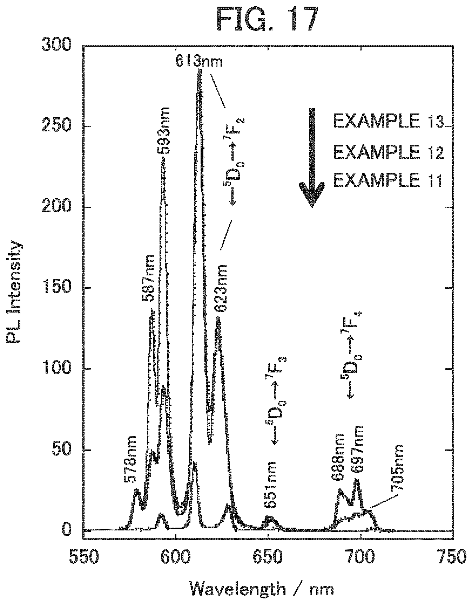

For particles of Examples 11 to 13, an excitation spectrum and a fluorescence spectrum were measured in the same manner as in Example 1. As an example of the results, fluorescence spectrum is shown in FIG. 17.

As a result, in all of Examples 11 to 13, an excitation peak (395 nm) and a light-emitting peak (613 nm) derived from an Eu(III) ion were observed. Furthermore, for particles of Examples 11 to 13, when internal quantum yields of the particles were obtained where excitation wavelength $\lambda_{ex}$ was 395 nm and light-emission wavelength $\lambda_{em}$ was 613 nm, the yield was 1.47 in Example 11, 5.39 in Example 12, and 6.70% in Example 13.

<Chemical composition (X-ray fluorescence analysis (XRF))>

For the particles of Examples 11 to 13 and Comparative Example 4, the X-ray fluorescence analysis (XRF) was carried out in the same manner as in Example 1. Results are shown in Tables 11 and 12.

[Table 11]

|  | Si (mol%) | O (mol%) | Eu (mol%) | C (mol%) | F (mol%) | Other element (mol%) |
|---|---|---|---|---|---|---|
| Example 11 | 31.7 | 65.3 | 0.391 | 2.36 | 0.214 | 0.035 |
| Example 12 | 31.6 | 64.0 | 0.860 | 2.18 | 1.35 | 0.010 |
| Example 13 | 31.1 | 61.4 | 1.50 | 2.01 | 3.99 | - |
| Comparative Example 4 | - | - | 23.7 | - | 76.3 | - |

[Table 12]

|  | Eu/Si (mol%) | F/Eu (mol%) | C/Si (mol%) | C/Eu (mol%) |
|---|---|---|---|---|
| Example 11 | 1.23 | 54.7 | 7.44 | 604 |
| Example 12 | 2.72 | 157 | 6.90 | 253 |
| Example 13 | 4.82 | 266 | 6.46 | 134 |
| Comparative Example 4 | - | 322 | - | - |

<Measurement of average particle size>

For particles of Examples 11 to 13, 100 or more particle diameters were measured using the field emission-type scanning electron microscope (FE-SEM), and an average particle size was calculated. Note here that for 300 particles, the longer diameter and the shorter diameter of each particle were measured, respectively, and "(longer diameter + shorter diameter) / 2" was defined as a particle diameter of each particle. An average value of the particle diameter of each particle (a value obtained by dividing the total values of the particle diameter of each particle by number of particles (300)) was defined as an average particle size. As a result, an average particle size of the particles of Example 11 was 140 nm, an average particle size of the particles of Example 12 was 149 nm, and an average particle size of the particles of Example 13 was 151 nm.

<Toxicity evaluation>

Toxicity evaluation was carried out using the particles of Examples 11 to 13 and Comparative Example 4 by the same method as in Example 1. As a result, Examples 11 to 13, in which crystal of a europium compound were formed, exhibited normal cell proliferation behavior and no cytotoxicity. On the other hand, in Comparative Example 4, since fluoride was unstable, this eluted into a cell culture solution, and was formed into Eu ion or Eu fluoride ion and reacted with cells directly, resulting in showing substantially the same level of toxicity as in Comparative Example 1. Note here that for the fluorescence property of the particles which have taken up by the cells, since the crystal did not elute into the cell culture solution, finally, after 48 hours of culture, high fluorescence intensity which was higher than the result of Comparative Example 4 was shown.

As mentioned above, it is shown that when composite particles having a chloride of the rare-earth element or a fluoride of the rare-earth element on a surface of a specific silicate-based base material is used, cells grow well, and the particles can be taken up by the cells and visualized. Since agglomeration of the rare-earth element as a light-emitting substance is suppressed, and the support amount of the rare-earth element is appropriate, the fluorescence intensity is high, and detection can be carried out with high sensitivity.

EXPLANATION OF REFERENCE NUMERALS

1 Silicate-based base material (spherical shape)

2 Rare-earth compound
21 Container
22 Ammonia water
23 Screw tube
24 Liquid surface of ammonia water
25 Stand

**Claims**

1. A light-emitting nanoparticle comprising a composite of a silicate-based base material and a rare-earth compound, the silicate-based base material comprising elemental silicon (Si) and elemental oxygen (O), wherein the silicate-based base material is a powder having an average particle diameter of 50 nm or more and 470 nm or less, and the rare-earth compound is a light-emitting substance,

   the rare-earth compound comprising at least one selected from a chloride of a rare-earth element and a fluoride of a rare-earth element,
   the silicate-based base material having a solid $^{29}$Si-NMR spectrum satisfying $Q_4/Q_3$ of 1.6 to 3.9 where $Q_4$ represents a peak area derived from $Si(OSi)_4$ and $Q_3$ represents a peak area derived from $HO\text{-}Si(OSi)_3$.

2. The light-emitting nanoparticle according to claim 1, wherein the rare-earth element is at least one selected from europium (Eu) and terbium (Tb).

3. The light-emitting nanoparticle according to claim 2, wherein the rare-earth compound includes at least one compound selected from compounds represented by the following chemical formulae (1), (3) and (4):

$$EuCl_x \qquad (1)$$

$$Eu(OH)_2Cl \qquad (3)$$

$$EuOCl \qquad (4)$$

   wherein, in the chemical formula (1), x is 0.05 or more and 5 or less;
   wherein *preferably* the rare-earth compound has a powder X-ray diffraction pattern having a first diffraction peak in a diffraction angle (2θ) range of 34.3° to 36.1° in which a half value width of the first diffraction peak of 1.8° or less, and/or having a second diffraction peak in a diffraction angle (2θ) range of 28.6° to 29.6° and a third diffraction peak in a diffraction angle (2θ) range of 36.8° to 38.4° in which a half value width of the second diffraction peak is 1.0° or less and a half value width of the third diffraction peak is 1.6° or less.

4. The light-emitting nanoparticle according to claim 2, wherein the rare-earth compound includes at least one selected from a compound represented by the following chemical formula (5) and a compound represented by the following chemical formula (6), and a mixed crystal of at least one compound selected from the compound represented by the chemical formula (5) and the compound represented by the chemical formula (6), and amorphous silica:

$$EuOF \qquad (5)$$

$$EuF_3 \qquad (6);$$

   wherein *preferably* the rare-earth compound has a powder X-ray diffraction pattern having a fourth diffraction peak in a diffraction angle (2θ) range of 26.6° to 28.6°, a fifth diffraction peak in a diffraction angle (2θ) range of 44.8° to 46.8°, and a sixth diffraction peak in a diffraction angle (2θ) range of 24.3° to 26.3°, in which a half value width of the fourth diffraction peak is 0.3° or less, a half value width of the fifth diffraction peak is 0.57° or less, and a half value width of the sixth diffraction peak is 0.22° or less, and/or having the fourth diffraction peak, the fifth diffraction peak, and a seventh diffraction peak in a the diffraction angle (2θ) range of 30.8° to 32.8°, in which a half value width is the fourth diffraction peak of 0.3° or less, a half value width of the fifth diffraction peak is 0.57° or less, and a half value width of the seventh diffraction peak is 0.38° or less.

5. The light-emitting nanoparticle according to any one of claims 1 to 4,

wherein the rare-earth element is on a surface of the silicate-based base material; and/or
wherein a percentage of a number of moles of the rare-earth element with respect to a total number of moles of the elemental silicon and the rare-earth element is 0.1 mol% or more and 7 mol% or less; and/or
wherein the silicate-based base material is a base material comprising silica or a silicate; and/or
wherein the silicate-based base material is an amorphous.

6. The light-emitting nanoparticle according to any one of claims 1 to 5, comprising a molecule containing elemental carbon on a surface.

7. Use of the light-emitting nanoparticle according to any one of claims 1 to 6 for bioimaging.

8. A cell detection method, comprising allowing the light-emitting nanoparticle according to any one of claims 1 to 6 to be taken up into a cell, irradiating the light-emitting nanoparticle with light, and observing the cell.

9. A medical device comprising a testing portion for carrying out testing of an internal cell, a diagnosis portion for carrying out diagnosis of the internal cell, and/or a treatment portion for carrying out treatment of the internal cell, and further comprising a light irradiating portion for introducing the light-emitting nanoparticle according to any one of claims 1 to 6 into an internal cell and irradiating the light-emitting nanoparticle with light, when the testing, the diagnosis, and/or the treatment are carried out.

10. A method for producing the composite of a silicate-based base material and a rare-earth compound as defined in any one of claims 1 to 6, the method comprising:

mixing a silicate-based base material and a raw material of a rare-earth compound with each other to carry out a solid-phase mechanochemical reaction,
wherein the silicate-based base material has a solid $^{29}$Si-NMR spectrum satisfying $Q_4/Q_3$ of 1.6 to 3.9 where $Q_4$ represents a peak area derived from $Si(OSi)_4$ and $Q_3$ represents a peak area derived from $HO\text{-}Si(OSi)_3$.

11. A method for producing a composite of a silicate-based base material and a rare-earth compound as defined in any one of claims 1 to 6, the method comprising:

dissolving a surface of the silicate-based base material in a basic atmosphere, and
reacting the silicate-based base material in which the surface is dissolved with the raw material of the rare-earth compound,
wherein the silicate-based base material has a solid $^{29}$Si-NMR spectrum satisfying $Q_4/_{Q3}$ of 1.6 to 3.9 where $Q_4$ represents a peak area derived from $Si(OSi)_4$ and $Q_3$ represents a peak area derived from $HO\text{-}Si(OSi)_3$.

12. A mixed crystal including at least one compound selected from a compound represented by the following chemical formula (5) and a compound represented by the following chemical formula (6), and amorphous silica, which is a powder having an average particle diameter of 50 nm or more and 470 nm or less, the mixed crystal having a powder X-ray diffraction pattern having a fourth diffraction peak in a diffraction angle (2θ) range of 26.6° to 28.6°, a fifth diffraction peak in a diffraction angle (2θ) range of 44.8° to 46.8°, and a sixth diffraction peak in a diffraction angle (2θ) range of 24.3° to 26.3°, in which a half value width of the fourth diffraction peak is 0.3° or less, a half value width of the fifth diffraction peak is 0.57° or less, and a half value width of the sixth diffraction peak is 0.22° or less, and/or having the fourth diffraction peak, the fifth diffraction peak, and a seventh diffraction peak in a diffraction angle (2θ) range of 30.8° to 32.8°, in which a half value width of the fourth diffraction peak is 0.3° or less, a half value width of the fifth diffraction peak is 0.57° or less, and a half value width of the seventh diffraction peak is 0.38° or less:

EuOF            (5)

$$EuF_3 \qquad (6).$$

**Patentansprüche**

1. Lichtemittierendes Nanopartikel, umfassend einen Verbundstoff aus einem Basismaterial auf Silikatbasis und einer Seltenerdverbindung, wobei das Basismaterial auf Silikatbasis elementares Silizium (Si) und elementaren Sauerstoff (O) umfasst, wobei das Basismaterial auf Silikatbasis ein Pulver mit einem durchschnittlichen Teilchendurchmesser von 50 nm oder mehr und 470 nm oder weniger ist und die Seltenerdverbindung eine lichtemittierende Substanz ist,

   wobei die Seltenerdverbindung mindestens eines; ausgewählt aus einem Chlorid eines Seltenerd-Elements und einem Fluorid eines Seltenerd-Elements, umfasst,
   das Basismaterial auf Silikatbasis ein festes $^{29}$Si-NMR-Spektrum aufweist, das $Q_4/Q_3$ von 1,6 bis 3,9 erfüllt, wobei $Q_4$ eine von $Si(OSi)_4$ abgeleitete Peakfläche darstellt und $Q_3$ eine von $HO-Si(OSi)_3$ abgeleitete Peakfläche darstellt.

2. Lichtemittierendes Nanopartikel gemäß Anspruch 1, wobei das Seltenerdelement mindestens eines ist, ausgewählt aus Europium (Eu) und Terbium (Tb).

3. Lichtemittierendes Nanopartikel gemäß Anspruch 2, wobei die Seltenerdverbindung mindestens eine Verbindung einschließt, die aus Verbindungen ausgewählt ist, die durch die folgenden chemischen Formeln (1), (3) und (4) dargestellt werden:

$$EuCl_x \qquad (1)$$

$$Eu(OH)_2Cl \qquad (3)$$

$$EuOCl \qquad (4)$$

   worin in der chemischen Formel (1) x 0,05 oder mehr und 5 oder weniger ist;
   wobei *vorzugsweise* die Seltenerdverbindung ein Pulverröntgenbeugungsmuster mit einem ersten Beugungspeak in einem Beugungswinkel (2θ)-Bereich von 34,3° bis 36,1° aufweist, bei dem die Halbwertsbreite des ersten Beugungspeaks 1,8° oder weniger beträgt, und/oder mit einem zweiten Beugungspeak in einem Beugungswinkel (2θ)-Bereich von 28,6° bis 29,6° und einem dritten Beugungspeak in einem Beugungswinkel (2θ)-Bereich von 36,8° bis 38,4°, bei dem eine Halbwertsbreite des zweiten Beugungspeaks 1,0° oder weniger und eine Halbwertsbreite des dritten Beugungspeaks 1,6° oder weniger beträgt.

4. Lichtemittierendes Nanopartikel gemäß Anspruch 2, wobei die Seltenerdverbindung mindestens eine Verbindung, ausgewählt aus einer Verbindung der folgenden chemischen Formel (5) und einer Verbindung der folgenden chemischen Formel (6), und einen Mischkristall aus mindestens einer Verbindung, ausgewählt aus der Verbindung der chemischen Formel (5) und der Verbindung der chemischen Formel (6), und amorphem Siliciumdioxid enthält:

$$EuOF \qquad (5)$$

$$EuF_3 \qquad (6);$$

   wobei *vorzugsweise* die Seltenerdverbindung ein PulverRöntgenbeugungsmuster mit einem vierten Beugungspeak in einem Beugungswinkel (2θ) im Bereich von 26,6° bis 28,6°, einem fünften Beugungspeak in einem Beugungswinkel (2θ) im Bereich von 44,8° bis 46,8°, und einen sechsten Beugungspeak in einem Beugungswinkel (2θ)-Bereich von 24,3° bis 26,3° aufweist, wobei eine Halbwertsbreite des vierten Beugungspeaks 0,3° oder weniger, eine Halbwertsbreite des fünften Beugungspeaks 0,57° oder weniger beträgt und eine Halbwertsbreite des sechsten Beugungspeaks 0,22° oder weniger beträgt, und/oder bei dem der vierte Beugungspeak, der fünfte Beugungspeak und ein siebter Beugungspeak in einem Beugungswinkel (2θ)-Bereich von 30,8° bis 32,8°, wobei die Halbwertsbreite des vierten Beugungspeaks 0,3° oder weniger beträgt, die Halbwertsbreite des fünften Beugungspeaks 0,57° oder weniger beträgt und die Halbwertsbreite des siebten Beugungspeaks 0,38° oder weniger beträgt.

**5.** Das lichtemittierende Nanopartikel gemäß einem der Ansprüche 1 bis 4,

wobei sich das Seltenerd-Element auf einer Oberfläche des Basismaterials auf Silikatbasis befindet; und/oder wobei der prozentuale Anteil einer Anzahl von Molen des Seltenerd-Elements in Bezug auf eine Gesamtanzahl von Molen des elementaren Siliciums und des Seltenerd-Elements 0,1 Mol-% oder mehr und 7 Mol-% oder weniger beträgt; und/oder wobei das Basismaterial auf Silikatbasis ein Basismaterial ist, das Siliziumdioxid oder ein Silikat enthält; und/oder wobei das Basismaterial auf Silikatbasis ein amorphes Material ist.

**6.** Lichtemittierendes Nanopartikel gemäß einem der Ansprüche 1 bis 5, umfassend ein Molekül, das elementaren Kohlenstoff auf einer Oberfläche enthält.

**7.** Verwendung des lichtemittierenden Nanopartikels nach einem der Ansprüche 1 bis 6 zum Bioimaging.

**8.** Zellnachweisverfahren, umfassend das Aufnehmen des lichtemittierenden Nanopartikels nach einem der Ansprüche 1 bis 6 in eine Zelle, das Bestrahlen des lichtemittierenden Nanopartikels mit Licht und das Beobachten der Zelle.

**9.** Medizinische Vorrichtung, die einen Testteil zur Durchführung eines Tests einer inneren Zelle, einen Diagnoseteil zur Durchführung einer Diagnose der inneren Zelle und/oder einen Behandlungsteil zur Durchführung einer Behandlung der inneren Zelle umfasst, und ferner umfassend einen Lichtbestrahlungsabschnitt zum Einführen des lichtemittierenden Nanopartikels gemäß einem der Ansprüche 1 bis 6 in eine interne Zelle und zum Bestrahlen des lichtemittierenden Nanopartikels mit Licht, wenn die Prüfung, die Diagnose und/oder die Behandlung durchgeführt werden.

**10.** Verfahren zur Herstellung eines Verbundwerkstoffs aus einem Basismaterial auf Silikatbasis und einer Seltenerdverbindung nach einem der Ansprüche 1 bis 6, wobei das Verfahren umfasst:

Mischen eines Basismaterials auf Silikatbasis und eines Rohmaterials aus einer Seltenerdverbindung zur Durchführung einer mechanochemischen Festphasenreaktion, wobei das Basismaterial auf Silikatbasis ein festes $^{29}$Si-NMR-Spektrum aufweist, das $Q_4/Q_3$ von 1,6 bis 3,9 erfüllt, wobei $Q_4$ eine von $Si(OSi)_4$ abgeleitete Peakfläche darstellt und $Q_3$ eine von $HO-Si(OSi)_3$ abgeleitete Peakfläche darstellt.

**11.** Verfahren zur Herstellung eines Komposits aus einem Basismaterial auf Silikatbasis und einer Seltenerdverbindung nach einem der Ansprüche 1 bis 6, wobei das Verfahren umfasst:

Auflösen einer Oberfläche des Basismaterials auf Silikatbasis in einer basischen Atmosphäre, und Reaktion des Basismaterials auf Silikatbasis, in dem die Oberfläche aufgelöst ist, mit dem Rohmaterial der Seltenerdverbindung, wobei das Basismaterial auf Silikatbasis ein festes $^{29}$Si-NMR-Spektrum aufweist, das $Q_4/Q_3$ von 1,6 bis 3,9 erfüllt, wobei $Q_4$ eine von $Si(OSi)_4$ abgeleitete Peakfläche darstellt und $Q_3$ eine von $HO-Si(OSi)_3$ abgeleitete Peakfläche darstellt.

**12.** Mischkristall, der mindestens eine Verbindung, die aus einer durch die folgende chemische Formel (5) dargestellten Verbindung und einer durch die folgende chemische Formel (6) dargestellten Verbindung ausgewählt ist, und amorphes Siliciumdioxid enthält, das ein Pulver mit einem durchschnittlichen Teilchendurchmesser von 50 nm oder mehr und 470 nm oder weniger ist, wobei der Mischkristall ein Pulverröntgenbeugungsmuster aufweist, das einen vierten Beugungspeak in einem Beugungswinkel ($2\theta$) im Bereich von 26,6° bis 28,6°, einen fünften Beugungspeak in einem Beugungswinkel ($2\theta$) im Bereich von 44,8° bis 46,8° und einem sechsten Beugungspeak in einem Beugungswinkel ($2\theta$)-Bereich von 24,3° bis 26,3° aufweist, wobei eine Halbwertsbreite des vierten Beugungspeaks 0,3° oder weniger beträgt, eine Halbwertsbreite des fünften Beugungspeaks 0,57° oder weniger beträgt und eine Halbwertsbreite des sechsten Beugungspeaks 0,22° oder weniger beträgt, und/oder bei dem der vierte Beugungspeak, der fünfte Beugungspeak und ein siebter Beugungspeak in einem Beugungswinkel ($2\theta$)-Bereich von 30,8° bis 32,8°, wobei eine Halbwertsbreite des vierten Beugungspeaks 0,3° oder weniger beträgt, eine Halbwertsbreite des fünften Beugungspeaks 0,57° oder weniger beträgt und eine Halbwertsbreite des siebten Beugungspeaks 0,38° oder weniger beträgt:

EuOF (5)

EuF$_3$       (6).

## Revendications

1. Une nanoparticule électroluminescente comprenant un composite d'un matériau de base à base de silicate et d'un composé de terre rare, le matériau de base à base de silicate comprenant du silicium élémentaire (Si) et de l'oxygène élémentaire (O), dans lequel le matériau de base à base de silicate est une poudre ayant un diamètre moyen de particule de 50 nm ou plus et de 470 nm ou moins, et le composé de terre rare est une substance électroluminescente,

   le composé de terre rare comprenant au moins un élément choisi parmi un chlorure de terre rare et un fluorure de terre rare,

   le matériau de base à base de silicate présente un spectre RMN-$^{29}$Si solide satisfaisant à Q$_4$/Q$_3$ de 1,6 à 3,9, où Q$_4$ représente une zone de pic dérivée de Si(OSi)$_4$ et Q$_3$ représente une zone de pic dérivée de HO-Si(OSi)$_3$.

2. La nanoparticule électroluminescente selon la revendication 1, dans laquelle l'élément de terre rare est au moins un élément choisi parmi l'europium (Eu) et le terbium (Tb).

3. la nanoparticule électroluminescente selon la revendication 2, dans laquelle le composé de terre rare comprend au moins un composé choisi parmi les composés représentés par les formules chimiques suivantes (1), (3) et (4) :

   EuCl$_x$       (1)

   Eu (OH)$_2$Cl       (3)

   EuOCl       (4)

   où, dans la formule chimique (1), x est égal ou supérieur à 0,05 et inférieur ou égal à 5 ;
   où, de *préférence,* le composé de terre rare présente un diagramme de diffraction des rayons X sur poudre comportant un premier pic de diffraction dans une plage d'angle de diffraction (2θ) de 34,3° à 36,1°, dans lequel la largeur de la demi-valeur du premier pic de diffraction est inférieure ou égale à 1,8°, et/ou comportant un deuxième pic de diffraction dans une plage d'angle de diffraction (2θ) de 28.6° à 29,6° et un troisième pic de diffraction dans une plage d'angle de diffraction (2θ) de 36,8° à 38,4° dans lequel une largeur de demi-valeur du deuxième pic de diffraction est de 1,0° ou moins et une largeur de demi-valeur du troisième pic de diffraction est de 1,6° ou moins.

4. La nanoparticule électroluminescente selon la revendication 2, dans laquelle le composé de terre rare comprend au moins un composé choisi parmi un composé représenté par la formule chimique suivante (5) et un composé représenté par la formule chimique suivante (6), et un cristal mixte d'au moins un composé choisi parmi le composé représenté par la formule chimique (5) et le composé représenté par la formule chimique (6), et de la silice amorphe :

   EuOF       (5)

   EuF$_3$       (6) ;

   où, de *préférence,* le composé de terre rare présente un diagramme de diffraction des rayons X sur poudre comportant un quatrième pic de diffraction dans une plage d'angle de diffraction (2θ) de 26,6° à 28,6°, un cinquième pic de diffraction dans une plage d'angle de diffraction (2θ) de 44.8° à 46,8°, et un sixième pic de diffraction dans une plage d'angle de diffraction (2θ) de 24,3° à 26,3°, dans lequel une largeur de demi-valeur du quatrième pic de diffraction est de 0,3° ou moins, une largeur de demi-valeur du cinquième pic de diffraction est de 0.57° ou moins, et une largeur de demi-valeur du sixième pic de diffraction est de 0,22° ou moins, et/ou ayant le quatrième pic de diffraction, le cinquième pic de diffraction, et un septième pic de diffraction dans une plage d'angle de diffraction (2θ) de 30.8° 32,8°, dans laquelle une largeur de demi-valeur du quatrième pic de diffraction est inférieure ou égale à 0,3°, une largeur de demi-valeur du cinquième pic de diffraction est inférieure ou égale à 0,57° et une largeur de demi-valeur du septième pic de diffraction est inférieure ou égale à 0,38°.

5. La nanoparticule électroluminescente selon l'une quelconque des revendications 1 à 4,

dans laquelle l'élément de terre rare se trouve sur une surface du matériau de base à base de silicate ; et/ou
dans laquelle le pourcentage du nombre de moles de l'élément de terre rare par rapport au nombre total de moles de silicium élémentaire et de l'élément de terre rare est égal ou supérieur à 0,1 % en moles et inférieur ou égal à 7 % en moles ; et/ou
dans laquelle le matériau de base à base de silicate est un matériau de base comprenant de la silice ou un silicate ; et/ou
dans laquelle le matériau de base à base de silicate est un matériau amorphe.

6. La nanoparticule électroluminescente selon l'une quelconque des revendications 1 à 5, comprenant une molécule contenant du carbone élémentaire sur une surface.

7. Utilisation de la nanoparticule électroluminescente selon l'une quelconque des revendications 1 à 6 pour la bioimagerie.

8. Procédé de détection cellulaire consistant à permettre à la nanoparticule électroluminescente selon l'une quelconque des revendications 1 à 6 d'être absorbée par une cellule, à irradier la nanoparticule électroluminescente avec de la lumière et à observer la cellule.

9. Dispositif médical comprenant une partie d'évaluation pour effectuer l'évaluation d'une cellule interne, une partie de diagnostic pour effectuer le diagnostic de la cellule interne et/ou une partie de traitement pour effectuer le traitement de la cellule interne, et

comprenant en outre une partie d'irradiation de la lumière pour introduire la nanoparticule électroluminescente selon l'une quelconque des revendications 1 à 6 dans une cellule interne et irradier la nanoparticule électroluminescente avec de la lumière, lorsque l'évaluation, le diagnostic et/ou le traitement sont effectués.

10. Procédé de fabrication du composite composé d'un matériau de base à base de silicate et d'un composé de terre rare tel que défini dans l'une quelconque des revendications 1 à 6, le procédé comprenant :

le mélange d'un matériau de base à base de silicate et d'une matière première constituée d'un composé de terre rare, afin d'effectuer une réaction mécano-chimique en phase solide,
dans lequel le matériau de base à base de silicate présente un spectre RMN- $^{29}$Si solide satisfaisant à $Q_4/_{Q3}$ de 1,6 à 3,9, où $Q_4$ représente une zone de pic dérivée de $Si(OSi)_4$ et $Q_3$ représente une zone de pic dérivée de HO-$Si(OSi)_3$.

11. Procédé de fabrication d'un composite composé d'un matériau de base à base de silicate et d'un composé de terre rare tel que défini dans l'une quelconque des revendications 1 à 6, le procédé comprenant :

dissoudre une surface du matériau de base à base de silicate dans une atmosphère basique, et
faire réagir le matériau de base à base de silicate dans lequel la surface est dissoute avec la matière première du composé de terre rare,
dans lequel le matériau de base à base de silicate présente un spectre RMN- $^{29}$Si solide satisfaisant à $Q_4/Q_3$ de 1,6 à 3,9, où $Q_4$ représente une zone de pic dérivée de $Si(OSi)_4$ et $Q_3$ représente une zone de pic dérivée de HO-$Si(OSi)_3$.

12. Cristal mixte comprenant au moins un composé choisi parmi un composé représenté par la formule chimique suivante (5) et un composé représenté par la formule chimique suivante (6), et de la silice amorphe, qui est une poudre dont le diamètre moyen des particules est supérieur ou égal à 50 nm et inférieur ou égal à 470 nm, le cristal mixte présentant un diagramme de diffraction des rayons X sur poudre comportant un quatrième pic de diffraction dans une plage d'angle de diffraction ($2\theta$) de 26,6° à 28,6°, un cinquième pic de diffraction dans une plage d'angle de diffraction ($2\theta$) de 44,8° à 46.8°, et un sixième pic de diffraction dans une plage d'angle de diffraction ($2\theta$) de 24,3° à 26,3°, dans lequel une largeur de demi-valeur du quatrième pic de diffraction est de 0,3° ou moins, une largeur de demi-valeur du cinquième pic de diffraction est de 0.57° ou moins, et une largeur de demi-valeur du sixième pic de diffraction est de 0,22° ou moins, et/ou ayant le quatrième pic de diffraction, le cinquième pic de diffraction, et un septième pic de diffraction dans une plage d'angle de diffraction ($2\theta$) de 30.8° à 32,8°, dans lequel une largeur de demi-valeur du quatrième pic de diffraction est de 0,3° ou moins, une largeur de demi-valeur du cinquième pic de diffraction est de 0,57°ou moins, et une largeur de demi-valeur du septième pic de diffraction est de à 0,38° ou moins :

$$EuOF \qquad (5)$$

$$EuF_3 \qquad (6).$$

FIG. 1

# FIG. 2

FIG. 3

# FIG. 4A

500 nm

# FIG. 4B

Ave.151 nm
Cv. 45 %

Particle size distribution / nm

Number of Particles

# FIG. 5

# FIG. 6

FIG. 7B

FLUORESCENCE SPECTRUM

EXAMPLE 3
EXAMPLE 2
EXAMPLE 1

615 nm $^5D_0 \rightarrow {}^7F_2$

594 nm $^5D_0 \rightarrow {}^7F_1$

591 nm

579 nm

650 nm $^5D_0 \rightarrow {}^7F_3$

698 nm $^5D_0 \rightarrow {}^7F_4$

PL Intensity

Wavelength / nm

FIG. 7A

EXCITATION SPECTRUM

EXAMPLE 3
EXAMPLE 2
EXAMPLE 1

395 nm $^7F_0 \rightarrow {}^5L_6$

362 nm $^7F_0 \rightarrow {}^5D_4$

376 nm
383 nm $^7F_0 \rightarrow {}^5G_4$

417 nm

465 nm $^7F_0 \rightarrow {}^5D_2$

526 nm $^7F_0 \rightarrow {}^5D_1$
534 nm

PL Intensity

Wavelength / nm

FIG. 8A

FIG. 8B

# FIG. 9A

# FIG. 9B

# FIG. 10

# FIG. 11

Legend:
- ○ EXAMPLE 1
- △ EXAMPLE 2
- □ EXAMPLE 3
- ◇ EXAMPLE 4
- ● COMPARATIVE EXAMPLE 1

Y-axis: Adhered cell density / cells·cm$^{-2}$
X-axis: Culture time / h

# FIG. 12

Legend:
- ○ EXAMPLE 1
- △ EXAMPLE 2
- □ EXAMPLE 3
- ◇ EXAMPLE 4
- ● COMPARATIVE EXAMPLE 1

Y-axis: PL Intensity
X-axis: Culture time / h

# FIG. 13

# FIG. 14

FIG. 15

## FIG. 16

# FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2010133887 A
- JP 2009190976 A
- JP 2009107106 A
- JP 2013014651 A
- WO 2009066548 A
- JP 2013057037 A
- WO 2005023961 A
- WO 2017170531 A

### Non-patent literature cited in the description

- **HASSAN SYED MUJTABA et al.** *Electrochimica Acta*, 2015, vol. 183, 160-164
- Selective molecular imaging of viable cancer cells with pH-activatable fluorescence probes. *Nature Medicine*, 2009, vol. 15, 104
- Quantum Dot Bioconjugates for Ultrasensitive Non-isotopic Detection.. *Science*, 2016, vol. 281
- Nucleic Acid-Passivated Semiconductor Nanocrystals: Biomolecular Templating of Form and Function.. *Accounts of Chemical Research*, 2010, vol. 43, 173
- Multimodal-Luminescence Core-Shell Nanocomposites for Targeted Imaging of Tumor Cells.. *Chem. Eur. J.*, 2009, vol. 15, 3577